# EUROPEAN PATENT SPECIFICATION

(11) **EP 4 081 196 B1**
(45) Date of publication and mention of the grant of the patent: **24.06.2026**
(21) Application number: 20839047.6
(22) Date of filing: 22.12.2020
(51) Int. Cl.: A61K 9/51, A61K 48/00, C12N 15/87, B82Y 5/00

(54) **GOLDEN LIPID NANOPARTICLES FOR GENE THERAPY**
GOLD-LIPIDNANOPARTIKEL FÜR DIE GENTHERAPIE
NANOPARTICULES LIPIDIQUES DORÉES POUR THÉRAPIE GÉNIQUE

(30) Priority: 23.12.2019 EP 19383183
(43) Date of publication of application: 02.11.2022
(73) Proprietor: Universidad del País Vasco/Euskal Herriko Unibertsitatea, 48940 Leioa, Vizcaya (ES)
(72) Inventor: DEL POZO RODRÍGUEZ, Ana, 01006 Vitoria-Gasteiz (ES); RODRÍGUEZ GASCÓN, Alicia, 01006 Vitoria-Gasteiz (ES); GÓMEZ AGUADO, Itziar, 01006 Vitoria-Gasteiz (ES); VICENTE PASCUAL, Mónica, 01006 Vitoria-Gasteiz (ES); SOLINÍS ASPIAZU, María Ángeles, 01006 Vitoria-Gasteiz (ES)
(74) Representative: ABG Intellectual Property Law, S.L.
(86) International application number: PCT/EP2020/087608
(87) International publication number: WO 2021/130215

(56) References cited:
- EP-B1- 2 460 516
- PENG WANG ET AL: "Genome Editing for Cancer Therapy: Delivery of Cas9 Protein/sgRNA Plasmid via a Gold Nanocluster/Lipid Core-Shell Nanocarrier", ADVANCED SCIENCE, vol. 4, no. 11, 7 September 2017 (2017-09-07), pages 1700175, XP055698823, ISSN: 2198-3844, DOI: 10.1002/advs.201700175
- PIETER R. CULLIS ET AL: "Lipid Nanoparticle Systems for Enabling Gene Therapies", MOLECULAR THERAPY : THE JOURNAL OF THE AMERICAN SOCIETY OF GENE THERAPY, vol. 25, no. 7, 1 July 2017 (2017-07-01), US, pages 1467 - 1475, XP055548712, ISSN: 1525-0016, DOI: 10.1016/j.ymthe.2017.03.013
- ALEX K. K. LEUNG ET AL: "Microfluidic Mixing: A General Method for Encapsulating Macromolecules in Lipid Nanoparticle Systems", JOURNAL OF PHYSICAL CHEMISTRY PART B, vol. 119, no. 28, 7 July 2015 (2015-07-07), US, pages 8698 - 8706, XP055551921, ISSN: 1520-6106, DOI: 10.1021/acs.jpcb.5b02891
- WON-KYU RHIM ET AL: "Lipid-Gold-Nanoparticle Hybrid-Based Gene Delivery", SMALL, vol. 4, no. 10, 1 October 2008 (2008-10-01), pages 1651 - 1655, XP055698906, ISSN: 1613-6810, DOI: 10.1002/smll.200800628
- GHASEM AMOABEDINY ET AL: "Overview of preparation methods of polymeric and lipid-based (niosome, solid lipid, liposome) nanoparticles: A comprehensive review", INTERNATIONAL JOURNAL OF POLYMERIC MATERIALS., vol. 67, no. 6, 28 August 2017 (2017-08-28), US, pages 383 - 400, XP055698955, ISSN: 0091-4037, DOI: 10.1080/00914037.2017.1332623
- BAYÓN-CORDERO LAURA ET AL: "Application of Solid Lipid Nanoparticles to Improve the Efficiency of Anticancer Drugs", NANOMATERIALS, vol. 9, no. 3, 22 March 2019 (2019-03-22), pages 474, XP055792268, Retrieved from the Internet <URL:https://www.ncbi.nlm.nih.gov/pmc/articles/PMC6474076/pdf/nanomaterials-09-00474.pdf> DOI: 10.3390/nano9030474

## Description

### Field of the Invention

The present invention relates to the field of gene therapy. In particular, the invention provides solid lipid nanoparticles (SLNs) useful as vectors for the transfection of nucleic acids, as well as methods for obtaining said SLNs and pharmaceutical compositions comprising said SLNs.

### Background

In the last few decades a wide range of reagents and techniques for transferring nucleic acids into cells for the purpose of modulating gene expression both *in vitro* and *in vivo* have been developed.

In particular, many genes involved in pathological processes have been identified and therefore gene therapy is viewed as a very promising therapeutic tool for reversing genetic alterations in said pathologies, which include for instance autosomal recessive diseases caused by defect of a single gene, e.g. cystic fibrosis, hemophilia, Fabry disease; autosomal dominant diseases; some cancers; HIV and other infectious diseases; inflammatory diseases; or ocular diseases such as Retinoschisis to name a few.

In order to develop a gene therapy product, not only the disease to be treated and the therapeutic nucleic acid to be administered are to be taken into account, but also the method of delivery of the nucleic acid, i.e. the administration system for the gene, plays a paramount role. The genetic vector employed must be capable of penetrating the cellular membrane of the target cell, as well as of protecting the nucleic acid against enzymatic degradation, and it ultimately must release the nucleic acid at the target site within the cell.

Administration systems for nucleic acids can be classified as viral or non-viral vectors.

Viral vectors are the most effective, but have large immunogenicity and oncogenicity problems. Another drawback is that they can only include small-sized genes.

Thus, much focus has been placed during the last years on developing nonviral vectors, which offer a safer and more versatile alternative. Amongst the various nonviral gene vectors, lipid-based systems have been proposed as promising platforms. These systems lack immunogenic proteins found in viruses and can generally condense nucleic acids robustly, and are further typically relatively straightforward to prepare. However, their transfection efficacy or the level of gene expression they provide is still generally substantially lower than that of viral vectors.

Distinct types of lipid-based transfection systems have been developed over the last couple of decades.

Liposomes were introduced as carriers for delivery of nucleic acids for gene therapy over two decades ago and to date still represent the most widely studied vectors for gene delivery. Liposomes used for gene delivery are typically nanometric and are defined by a spherical vesicle with an aqueous internal cavity enclosed by a lipid bilayer membrane. However, these systems are not devoid of stability and efficacy issues.

Solid lipid nanoparticles (SLNs) were more recently developed with the aim of, amongst other things, addressing the above issues underlying liposome gene transfection. Whilst sharing a lipid component, SLNs are structurally distant from liposomes. SLNs are spherical particles which possess a solid lipid core matrix which is stabilized by surfactants. Depending on the selection of the lipid and surfactant components, as well as on the method of preparation, SLNs may be used for harboring both hydrophilic and hydrophobic drugs. European patent EP 2460516 B1, by the present inventors, describes SLNs suitable as gene therapy vectors.

Despite recent advances, improvements over existing systems are always needed, and in particular, gene delivery platforms which further the capabilities of prior art vectors in terms of transfection and expression efficacies and/or in terms of the physical properties of the vectors are greatly desired.

### Brief Description of the Invention

The present inventors have now found that the use of gold with SLNs is of great advantage in terms of transfection and expression efficacies and/or in terms of the physical properties of the SLN.

Whilst the use of gold has been proposed in the past in the context of liposomes, generally with the aim of providing robustness and thus stability to their aqueous cores, it is as far as the inventors are aware of never been suggested for SLNs, which, contrary to liposomes, require no core stabilization as they already possess solid lipid cores. However, as stated above, it has surprisingly been found that gold may still nevertheless be used advantageously in SLN vectors.

Thus, in a first aspect, the invention relates to a SLN comprising:
- a lipid solid at room temperature at the core;
- a cationic surfactant;
- a non-ionic surfactant;
- gold, wherein the gold is absorbed onto the surface of the SLN or it is found at a hydrophilic phase of the SLN surrounding the lipid core of the SLN; and
- a nucleic acid.

The present inventors have also surprisingly found that the method by which the SLN of the first aspect of the invention is prepared can have an impact on the above mentioned transfection and expression efficacies and/or the physical properties of the SLN, thus allowing for great versatility to target different and varied types of cells.

Therefore, the second aspect of the invention refers to a process for the preparation of a SLN according to the first aspect of the invention (hereinafter referred to as "Process Au"), the process comprising:
a) Preparing a suspension of precursor SLN, said precursor SLN comprising:
   - a lipid solid at room temperature at the core of the precursor SLN;
   - a cationic surfactant; and
   - a non-ionic surfactant;
b) Preparing a solution comprising:
   - a nucleic acid; and
   - gold;
c) Mixing the suspension of precursor SLN obtained in step (a) with the solution obtained in step (b).

Similarly, the third aspect of the invention refers to another process for the preparation of a SLN according to the first aspect of the invention (hereinafter referred to as "Process Oro"), the process comprising:
a) Preparing a suspension of precursor gold SLN, said precursor gold SLN comprising:
   - a lipid solid at room temperature at the core;
   - a cationic surfactant;
   - a non-ionic surfactant; and
   - gold;
b) Preparing a solution comprising a nucleic acid;
c) Mixing the suspension of precursor gold SLN obtained in step (a) with the solution obtained in step (b).

Likewise, the invention also provides a pharmaceutical composition comprising the SLN of the first aspect of the invention.

In another aspect, the invention relates to the SLN of the first aspect of the invention for use as a medicament, and more particularly for use in gene therapy.

### Description of the Drawings

Figure 1 depicts a plurality of gold nanoparticles obtained according to the Turkevich method described in Example 1 of the present application.
Figure 2 illustrates a plurality of SLNs according to the present invention. In particular, it is a TEM image of a plurality of SLN_Au-P-HA nanoparticles according to Example 9 of the present application.
Figure 3 shows immunofluorescence staining images of corneal tissue after topical treatment with a comparative formulation (a) SLN-P-HA-mRNA; or with a formulation according to the present invention (b) SLN_Au-P-HA-mRNA, (c) SLN_Au-P-DX-DNA or (d) SLN_oro-P-DX-DNA. Encircled areas identify GFP fluorescence.

### Detailed Description of the Invention

In the context of the present invention, the term "solid lipid nanoparticle" or "SLN" refers to a structure comprising a lipophilic lipid core surrounded by a hydrophilic phase encapsulating the core.

In particular, the invention is directed to a plurality of SLNs according to the invention. Such plurality of SLNs is herein also referred to as the "system" of the invention. Embodiments described herein for the SLNs of the invention are understood to apply equally to the plurality of SLNs. The term "plurality" refers to any number of SLNs typically employed in practice for storage or administration to a subject, and can range from as few as 10² to any number reasonable for the intended purpose, e.g. a therapeutically effective but non-toxic amount of SLNs.

In an embodiment, the SLN of the invention has a size comprised between 1 and 1000 nm, more particularly between 100 and 800 nm.

Similarly, the SLN system of the invention preferably presents average SLN sizes comprised between 1 and 1000 nm, more particularly between 100 and 800 nm. "Average size" is understood as the average size of the plurality of SLNs comprising both the lipophilic phase at the core of the SLN, the hydrophilic phase surrounding said core, as well as any component of the SLN adsorbed thereon. It is in some cases still possible to find isolated populations, typically aggregates of SLNs, ranging up to about 10 µm within the system of the invention, however these will still fall within the scope of the invention so long as the average size of the system is within the above stated ranges.

The average sizes of the SLNs in these systems can be measured by standard methods known to the person skilled in the art, such as by microscopy (e.g. e.g. optical microscopy, negative stain electron microscopy, transmission electron microscopy (TEM), cryo-TEM, scanning electron microscopy (SEM), confocal microscopy and scanning probe microscopy), diffraction and scattering techniques (laser light scattering and photon correlation spectroscopy) and hydrodynamic techniques (field flow fractionation, gel permeation chromatography, ultracentrifugation and centrifugal sedimentation). Preferred techniques are dynamic light scattering (DLS) and more specifically photon correlation spectroscopy. Sizes measured by DLS correspond to the diameter of the sphere that diffuses at the same speed as the particle being measured.

The SLN system of the invention preferably presents a polydispersity index (PDI) of 0.7 or lower, more particularly of between 0.2 and 0.5. The term "polydispersity" (or "dispersity" as recommended by IUPAC) is used to describe the distribution of size populations within a given sample and to thus observe the degree of non-uniformity in sizes of said sample. The PDI is dimensionless, and numerical values range from 0.0 (for a perfectly uniform sample with respect to the particle size) to 1.0 (for a highly polydisperse sample with multiple particle size populations). PDIs can be measured by the standard methods known to the person skilled in the art mentioned above for the measurement of SLN sizes.

In the context of the present invention, the methods used for the determination of size and PDI parameters are preferably those defined in ISO standards 13321:1996 E and ISO 22412:2008 (Edition 1 in both cases). These measurements can be carried out employing appropriate Malvern Instruments Ltd. Zetasizer equipment (see for instance measurement as described in the Zetasizer Nano User Manual, MAN0317, Issue 5.0, August 2009 for a Nano ZS series device).

The present inventors have now surprisingly found that the incorporation of gold in SLNs is capable of reducing the size and polydispersity thereof. The possibility of reducing these crucial physical parameters, and with it to improve their tunability, are of paramount importance in the field of the invention, as reviewed by Danaei et al., Pharmaceutics. 2018 Jun; 10(2): 57. The inventors have further observed that this effect is particularly pronounced when the SLN comprises a glycosaminoglycan (GAG), preferably hyaluronic acid, as polysaccharide.

In addition, the SLNs of the invention show a zeta potential that can vary from -50 mV to +80 mV, and more particularly from +5 mV to +50 mV. Zeta potentials are most typically measured by Laser Doppler Velocimetry, or by Laser Doppler Electrophoresis, a technique which combines Electrophoresis and Laser Doppler Velocimetry. This technique measures how fast a particle moves in a liquid when an electrical field is applied - i.e. its velocity. In the context of the present invention, zeta potential is measured according to ISO standard ISO 13099-2:2012. These measurements can be carried out employing appropriate Malvern Instruments Ltd. Zetasizer equipment, such as Zetasizer Nano/Pro/Ultra.

In a preferred embodiment, the SLN further comprises a positively charged peptide.

In another preferred embodiment, the SLN further comprises a polysaccharide.

In another preferred embodiment, the SLN further comprises both a polysaccharide and a positively charged peptide.

Although already implied by the structure of the SLN, the SLN of the present invention is neither a liposome nor a liposome-based system, in particular it is not a system comprising an aqueous core.

The different components of the SLNs of the invention are described in detail below.

### Lipid component

The SLN of the present invention comprises at least one lipid solid at room temperature forming part of the SLN core.

In the context of the present invention, "lipid solid at room temperature" refers to a lipid which is maintained as a solid under 45°C, and which can be saturated or unsaturated. Said definition includes triglycerides (for example tristearin), mono- or diglycerides (for example Imwitor^{®}), fatty acids (for example stearic acid), steroids (for example cholesterol) and waxes (for example cetyl palmitate).

In a particular embodiment, the lipid solid at room temperature is selected from acylglycerides, saturated fatty acids with a chain of at least 10 carbon atoms or derivatives thereof and mixtures thereof.

The acylglycerides include both monoglycerides, diacylglycerides, triacylglycerides as well as mixtures thereof. In a preferred embodiment the acylglycerides are selected from glyceryl palmitostearate (Precirol^{®} ATO5), glyceryl monostearate (Imwitor^{®}900) and glyceryl behenate (Compritol^{®} 888ATO).

In a particular embodiment, the fatty acids are saturated and have a chain of at least 10 carbon atoms. Likewise, derivatives of these fatty acids, being understood as those compounds produced as a result of the reaction of the acid group with alcohols or amines such as, for example, the esters or amides of said fatty acids, can be used. Similarly, those fatty acids, their esters or their amides having hydroxyl groups as substituents of the hydrocarbon chain are included in the definition of fatty acid derivatives.

In a preferred embodiment, glyceryl palmitostearate (Precirol^{®} ATO5) is used as fatty acid derivative.

In a preferred embodiment, glyceryl palmitostearate (Precirol^{®} ATO5) or glyceryl monostearate is used as lipid solid at room temperature. More preferably, the lipid solid at room temperature is glyceryl palmitostearate.

In another particular embodiment, the SLNs of the invention further comprise another lipid component, specifically a lipid liquid at temperature less than 45°C, which can be saturated or unsaturated. The lipid liquid at room temperature is selected from unsaturated or saturated fatty acid esters, oils, fatty acids and triglycerides having a chain with less than 10 carbon atoms, and their mixtures (for example Miglyol^{®}, soybean oil, isopropyl myristate, castor oil). In a preferred embodiment, Mygliol 212 is used as the liquid lipid.

### Cationic surfactant

The term "cationic surfactant" as used herein refers to a compound having a hydrophobic part and a hydrophilic part which forms positively charged ions in a solution, typically a positively charged head group bound to a hydrophobic tail. The hydrophobic part becomes embedded in the lipidic core of the SLN, and the hydrophilic cationic part surrounds and encases said core thus forming the hydrophilic phase of the SLN.

This component provides a positive charge to the SLN of the invention which facilitates its absorption through cationic biological environments or its adsorption thereon.

In an embodiment, in the plurality of SLNs of the present invention, at least 50%, preferably at least 70%, more preferably at least 90% of the SLNs do not comprise cationic surfactant adsorbed onto the surface of the SLN or forming part of any complex adsorbed onto the surface of the SLN. The upper percentage limit is established based on the precision of the process for preparing the SLN, and can be up to 90%, up to 95%, up to 99% or up to 99.9%.

In a particular embodiment of the invention, the cationic surfactant is selected from linearly or cyclically structured primary, secondary, tertiary and quaternary ammonium salts, and mixtures thereof, such as for example pyridine, piperazine salts.

Likewise, derivatives of these ammonium salts can be used. Ammonium salts derivatives is understood as those salts incorporating at least two either primary, secondary, tertiary and/or quaternary amino groups, such as for example, guanidine, piperazine and imidazole salts in the same structure. This definition would also comprise amino acid salts, such as for example, lysine, arginine, ornithine or tryptophan salts. Likewise, this definition would encompass those ammonium salts in which the positive charge, instead of on the nitrogen atom, is on a phosphorus atom, such as for example, ditetradecyl (trimethylethylphosphonio) methylphosphonate iodide, ditetradecyl (butyldimethylphosphonio) methylphosphonate iodide, ditetradecyl (dimethylisopropylphosphonio) methylphosphonate iodide) or arsenic (ditetradecyl (trimethylarsonio) methylphosphonate iodide, dioleyl (trimethylphosphonio) methylphosphonate iodide).

In a preferred embodiment of the present invention, the ammonium salts are tetraalkylammonium salts, alkylbenzyl dimethyl ammonium salts or heterocyclic ammonium salts, more preferably are cetyltrimethylammonium bromide (CTAB), N-[1-(2,3-dioleoyloxy)propyl]-N,N,N-trimethylammonium chloride (DOTAP), or N-[1-(2,3-dioleyloxy)propyl]-N,N,N-trimethylammonium chloride (DOTMA) or 1,2-dioleoyl-3-dimethylammonium-propane (DODAP).

In an embodiment, the SLN of the invention comprises at least two cationic surfactants, such as two cationic surfactants. More particularly, the two cationic surfactants are ammonium salts as described above, and most particularly they are selected from DOTAP, DODAP, DOTMA or CTAB.

### Non-ionic surfactant

The SLNs of the invention additionally comprise a non-ionic surfactant the main functions of which are to control the particle size of and confer stability to the SLN, preventing the rupture of the latter and the formation of aggregates thereof.

The term "non-ionic surfactant" as used herein refers to a compound having a hydrophobic part and a hydrophilic part which allows producing an emulsion.

In a particular embodiment of the present invention the non-ionic surfactant is selected from polyethoxylated sorbitan-fatty acid esters, such as polysorbates, polyethylene glycol copolymers and polypropylene glycol copolymers, such as for example, Tween, Span, Poloxamer. Preferably, the non-ionic surfactant is a polyethoxylated sorbitan-fatty acid ester, more preferably it is a polysorbate, in particular polyoxyethylene sorbitan oleate, and most particularly Tween/Polysorbate 80. In another preferred embodiment, the non-ionic surfactant is a poloxamer.

In an embodiment, the SLN of the invention comprises at least two non-ionic surfactants, such as two non-ionic surfactants. More particularly, the two non-ionic surfactants may be a polysorbate and a poloxamer as described above.

### Gold

The gold in the SLN of the present invention may be in the form of a nanoparticle or a nanostructure selected from nano-rods, nano-ellipsoids, nano-prisms, nano-stars, nano-cages and nano-shells. In a preferred embodiment, the gold is in the form of a nanoparticle. These gold forms typically have a size comprised between 1 and 100 nm, more preferably between 2 and 50 nm, such as between 2 and 8 nm, or such as between 15 and 25 nm.

The plurality of SLNs of the invention presents also a plurality of gold nanoparticles or nanostructures with average sizes comprised between 1 and 100 nm, more preferably between 2 and 50 nm, such as between 2 and 8 nm, or such as between 15 and 25 nm. "Average size" is understood as the average size of the plurality of gold nanoparticles or nanostructures. It is still possible to find isolated gold nanoparticles or nanostructures of a size greater than that specified above, so long as the average size of the plurality is within the above stated ranges.

The gold plurality typically presents a polydispersity index (PDI) of 0.7 or lower, more particularly of between 0.1 and 0.4. The term "polydispersity" has the meaning described above.

In addition, the gold nanoparticles or nanostructures typically show a zeta potential that can vary from -60 mV to -20 mV, and more particularly varies from -50 mV to -40 mV.

Sizes, PDIs and zeta potentials of gold nanoparticles or nanostructures are measured in the same manner as was described above for the SLNs of the invention.

This component can be part of the SLN structure or can be adsorbed on the surface thereof. When the gold is part of the SLN structure, it is found in the hydrophilic phase of the SLN, together with the cationic surfactant and the non-ionic surfactant.

It has surprisingly been found that the incorporation of gold, especially gold nanoparticles, typically negatively charged, into a SLN, and in particular into the hydrophilic phase of a SLN or adsorbed thereon, is not detrimental to cellular penetration even though a greater repulsion/lower affinity towards the also negatively charged cellular membrane would in theory be expected. In fact, nucleic acid transfection and expression levels can be significantly boosted with the incorporation of the gold nanoparticle or nanostructure.

In an embodiment, in the plurality of SLNs of the present invention, at least 50%, preferably at least 70%, more preferably at least 90% of the SLNs which comprise gold comprise the gold at the hydrophilic phase of the SLN or adsorbed thereon. The upper percentage limit is established based on the precision of the process for preparing the SLN, and can be up to 90%, up to 95%, up to 99% or up to 99.9%.

In an embodiment, in the plurality of SLNs of the present invention, at least 50%, preferably at least 70%, more preferably at least 90% of the SLNs which comprise gold comprise the gold at the hydrophilic phase of the SLN. The upper percentage limit is established based on the precision of the process for preparing the SLN, and can be up to 90%, up to 95%, up to 99% or up to 99.9%.

In an embodiment, in the plurality of SLNs of the present invention, at least 50%, preferably at least 70%, more preferably at least 90% of the SLNs which comprise gold comprise the gold adsorbed onto the hydrophilic phase of the SLN. The upper percentage limit is established based on the precision of the process for preparing the SLN, and can be up to 90%, up to 95%, up to 99% or up to 99.9%.

In the SLN of the present invention, the gold is not covalently bound to the nucleic acid. In the plurality of SLNs, at least 50%, preferably at least 70%, more preferably at least 90% of the gold nanoparticles or nanostructures are not covalently bound to the nucleic acid.

In the context of the present invention at least 50%, preferably at least 70%, more preferably at least 90% of the SLNs which comprise gold do not comprise it at the lipidic core of the SLN. The upper percentage limit is established based on the precision of the process for preparing the SLN, and can be up to 90%, up to 95%, up to 99% or up to 99.9%.

### Nucleic acid

The SLNs of the invention can harbor nucleic acids and deliver them into cells, where they are released to exert their therapeutic action.

The nucleic acid can be part of the SLN structure or can be adsorbed on the surface thereof. When the nucleic acid is part of the SLN structure, it is found in the hydrophilic phase of the SLN, together with the cationic surfactant and the non-ionic surfactant.

As used herein, the term "nucleic acid" refers to any chain of two or more nucleotides covalently bonded together, including, without limitation, ribonucleic acid (RNA), e.g. i) RNAs involved in protein synthesis, such as messenger RNA (mRNA), e.g. Zinc-finger nuclease (ZFN)-encoding mRNA, Transcription activator-like effector nuclease (TALEN)-encoding mRNA, or CRISPR/Cas9-encoding mRNA, transfer RNA (tRNA), transfer-messenger RNA (tmRNA), Ribosomal RNA (rRNA); ii) RNAs involved in post-transcriptional modification or DNA replication, such as small nuclear RNA (snRNA), small nucleolar RNA (snoRNA), SmY RNA, small Cajal body-specific RNA (scaRNA), Guide RNA (gRNA), Ribonuclease P (RNase P), Ribonuclease MRP (RNase MRP), Y RNA, Telomerase RNA Component (TERC), Spliced Leader RNA (SL RNA); iii) regulatory RNAs, such as Antisense RNA (aRNA or asRNA), Cis-natural antisense transcript (cis-NAT), CRISPR RNA (crRNA), Long noncoding RNA (lncRNA), MicroRNA (miRNA), Piwi-interacting RNA (piRNA), Small interfering RNA (siRNA), Short hairpin RNA (shRNA), Trans-acting siRNA (tasiRNA), Repeat associated siRNA (rasiRNA), 7SK RNA, Enhancer RNA (eRNA); Deoxyribonucleic acid (DNA), such as plasmids or such as Zinc-finger nuclease (ZFN)-encoding DNA, Transcription activator-like effector nuclease (TALEN)-encoding DNA, or CRISPR/Cas9-encoding DNA, Genomic DNA (gDNA), complementary DNA (cDNA), Mitochondrial DNA (mtDNA), scaffold/matrix attachment region DNA (S/MAR DNA), Antisense DNA (asDNA); or DNA/RNA hybrids. Preferred RNAs are mRNA, siRNAs, miRNA and asRNA, most preferably mRNA. Preferred DNAs are plasmid DNA and asDNA. In a preferred embodiment, the nucleic acid is a mRNA or a DNA plasmid. In a particular embodiment, the nucleic acid is a mRNA. In another particular embodiment, the nucleic acid is a DNA plasmid.

A preferred class of nucleic acids are antisense nucleic acids, i.e. nucleic acids that can bind to and inactivate mRNA produced from a gene in a subject.

The nucleic acid may be composed of naturally occurring nucleotides, or it may comprise synthetic nucleotides (also known as nucleotide analogues). Examples of the latter nucleotides are 2'-O-methyl nucleotides, locked nucleotides (LNA), bridged nucleotides (BNA), morpholinos, and peptide nucleotides (PNA).

The nucleic acids may be linear (e.g. mRNA), circular (e.g. DNA plasmids) or branched. Preferred linear nucleic acids are oligonucleotides, i.e. nucleic acids made up of 6 to 200, such as of 10 to 30, nucleotides or nucleotide pairs (if oligonucleotide double stranded).

The nucleic acids may be single-stranded, or partially or completely double-stranded. When double stranded, the nucleic acid may adopt an A-, B-, Z- or P-configuration. In a preferred embodiment of the invention, the nucleic acid is single-stranded, and is more preferably single-stranded RNA. In another preferred embodiment of the invention, the nucleic acid is double-stranded, and is more preferably double-stranded DNA.

### Polysaccharide

In some embodiments, the SLN of the present invention additionally comprises a polysaccharide.

This component can be part of the SLN structure or can be adsorbed on the surface thereof. When the polysaccharide is part of the SLN structure, it is found in the hydrophilic phase of the SLN, together with the cationic surfactant and the non-ionic surfactant.

The polysaccharide can form a complex with the nucleic acid by means of ionic interaction. Said complex is contacted with the previously formed precursor SLNs, such that the complex formed by the polysaccharide and the active ingredient is adsorbed on the surface of said nanoparticles or incorporated in the hydrophilic phase of the SLN.

According to a particular embodiment, the polysaccharide comprises the binding of at least three monosaccharides. According to another embodiment, the polysaccharide is not a lipopolysaccharide.

In an embodiment, the polysaccharide is selected from chitosan, dextran, carrageenan, colominic acid, xanthan, cyclodextrins, glycosaminoglycans and mixtures thereof. Preferably, the polysaccharide is dextran or a glycosaminoglycan. Examples of glycosaminoglycans are heparin, heparan sulfate, chondroitin sulfate, dermatan sulfate, keratan sulfate, and hyaluronic acid. Preferably, the glycosaminoglycan is hyaluronic acid. In a particular embodiment, the polysaccharide is dextran. In another particular embodiment, the polysaccharide is hyaluronic acid.

### Positively charged peptide

In some embodiments, the SLN of the present invention additionally comprises a positively charged peptide, this is, a peptide which becomes ionized in solution to a net positive charge. Preferably, the positively charged peptide is one which is positively charged when subjected to a pH of 10 or lower, such to a pH selected from 2.5 to 10, preferably when subjected to physiologic pH (pH 7.4). The positively charged peptide is also herein referred to as "peptide with a net positive charge".

The positively charged peptide can be part of the SLN structure or can be adsorbed on the surface thereof. When the positively charged peptide is part of the structure of the SLN, it is found in the hydrophilic phase of the SLN, together with the cationic surfactant and the non-ionic surfactant.

Alternatively, the positively charged peptide can form a complex together with the polysaccharide and/or the nucleic acid by means of ionic interaction. Said complex is contacted with the previously formed precursor SLNs, such that the complex formed by the positively charged peptide and the polysaccharide and/or the nucleic acid is adsorbed on the surface of said nanoparticles.

In a particular embodiment of the present invention, the positively charged peptide is selected from nuclear signaling peptides and mitochondrial signaling peptides, arginylglycylaspartic acid (RGD) peptides and cell-penetrating peptides (CPP). Said peptide is preferably selected from protamines and histones, and is most preferably protamine.

In an aspect, the invention is directed to a suspension comprising the plurality of SLNs of the invention as described in any embodiment above dispersed in a suspension medium, such as in an aqueous suspension medium, e.g. water or saline. The suspension may comprise from 1 to 50 % by weight, preferably from 10% to 20% by weight, of SLNs of the invention with respect to the total weight of the suspension. The suspension may comprise additional components such as pharmaceutically acceptable excipients as described further below. In an embodiment, the suspension consists of SLNs of the invention and suspension medium.

In an embodiment, the SLNs of the present invention are in lyophilised form. This form is suitable for storage of the SLNs and reconstitution thereof, such as reconstitution into a suspension as described above, at a later time. The inventors have found that such procedure does not substantially alter the transfection capabilities of the SLNs of the present invention.

In another aspect, the invention relates to different processes for the preparation of an SLN or plurality thereof as described in any of the above embodiments. Specifically, two types of processes are provided herein and referred to as "Process Au" and "Process Oro". The present inventors have unexpectedly found that the SLNs obtained by these methods (but comprising the same components) differ in their physical properties and biological activity. This allows fine tuning the vectors of the present invention to different biological contexts, such as to target cells of varying characteristics.

### Process Au

In an embodiment, the process of the invention comprises the steps of:
a) preparing a suspension of precursor SLN, said precursor SLN comprising:
   - a lipid solid at room temperature at the core;
   - a cationic surfactant; and
   - a non-ionic surfactant;
b) preparing a solution comprising:
   - a nucleic acid;
   - gold; and
c) mixing the suspension of precursor SLN obtained in step
   a) with the solution obtained in step b).

Step a) comprises preparing a suspension of precursor SLN, such as by methods well known to the person skilled in the art. In an embodiment, step a) comprises:
(i) preparing a solution comprising the lipid solid at room temperature in an organic solvent;
(ii) preparing an aqueous solution comprising the cationic surfactant and the non-ionic surfactant;
(iii) adding the aqueous solution (ii) to the organic solution (i), and subjecting the resulting mixture to stirring until obtaining an emulsion; and
(iv) evaporating the organic solvent.

Step (i) comprises preparing a solution comprising the lipid solid at room temperature in an organic solvent, such as by methods well known to the person skilled in the art, such as by dissolving the lipid solid at room temperature in an organic solvent. In an embodiment, the proportion of the lipid component is at least 0.1%, preferably between 0.1 and 40%, by weight with respect to the total weight of the organic solution.

The choice of the organic solvent depends to a large extent on both the lipid component and, where appropriate, on the nucleic acid to be incorporated. Regardless of the above, the use of pharmaceutically acceptable organic solvents and the use of the smallest possible amount are preferred due to the fact that it will have to subsequently be removed in the final step of the method which leads to the precursor SLNs.

In a particular embodiment of the invention, the organic solvent is selected from dichloromethane, acetone, chloroform, and is more preferably dichloromethane. The proportion of the organic solvent can range between 1 and 60%, preferably between 10 and 30%, by weight with respect to the total weight of the emulsion obtained at step (iii).

When a lipid liquid at room temperature is to be employed, it may be added to the organic solution at step (i).

Step (ii) comprises preparing an aqueous solution comprising the cationic surfactant and the non-ionic surfactant, such as by methods well known to the person skilled in the art, such as by dissolving the cationic surfactant and the non-ionic surfactant in an aqueous solvent.

At step (iii), the aqueous phase is added to the organic (lipidic) phase. This specific order of addition is preferred. The resulting mixture is subjected to a rigorous stirring until obtaining an emulsion. A preferred form of stirring is sonication. The emulsion obtained in this step is an oil in water emulsion.

Subsequently, at step (iv) the organic solvent is evaporated by means of any method known by a person skilled in the art. In a particular embodiment, the organic solvent evaporation step is carried out by keeping the emulsion under mechanical stirring for at least five minutes, subsequently subjecting it to vacuum for at least five minutes. After removing the organic solvent, the lipid phase solidifies, thus obtaining the precursor SLNs in the form of an aqueous suspension.

In a particular embodiment, step a) further comprises a step v) of isolating the precursor SLNs from the precursor SLN suspension obtained at step iv), such as by centrifugation. In a more particular embodiment, the isolation step includes cooling the precursor SLN suspension to a temperature comprised between 4 and 8°C and subsequently filtering the precursor SLNs by centrifugation.

In an embodiment, the precursor SLNs, with or without isolating, may be lyophilized. The lyophilized SLNs may be stored, and resuspended in any suspension medium at a later time for use in step c) of the process of the invention.

In another embodiment, step a) comprises:
(i) melting the lipid solid at room temperature,
(ii) preparing an aqueous solution comprising the cationic surfactant and the non-ionic surfactant,
(iii) adding the aqueous solution (ii) to the melted lipid (i), and subjecting the resulting mixture to stirring until obtaining an emulsion; and
(iv) optionally, subjecting the emulsion (iii) to a homogenization process with a pressure of at least 30 psi.

At step (i), the lipid solid at room temperature is melted at a temperature greater than its melting point. When a lipid liquid at room temperature is to be employed, it may be added to the melted lipid at this stage.

Step (ii) comprises preparing an aqueous solution comprising the cationic surfactant and the non-ionic surfactant, such as by methods well known to the person skilled in the art, such as by dissolving the cationic surfactant and the non-ionic surfactant in an aqueous solvent.

In an embodiment, step (ii) comprises heating the aqueous solution to a temperature equal to or greater than the melting point of the lipid solid at room temperature of step (i). For instance, the lipid solid at room temperature in step (i) and the aqueous solution in step (ii) are independently heated to the same temperature.

At step (iii), the aqueous phase is added to the organic (lipidic) phase. This specific order of addition is preferred. Preferably, the addition is carried out while the aqueous phase and the lipidic phase are at a temperature equal to or greater than the melting point of the lipid solid at room temperature of step (i).

The resulting mixture is subjected to stirring. A preferred form of stirring is sonication. The stirring is preferably performed at a temperature equal to or greater than the melting point of the lipid solid at room temperature of step (i). The emulsion obtained in this step is an oil in water emulsion.

The obtained emulsion may be allowed to cool down to below the melting point of the lipid solid at room temperature of step (i). In this manner, said lipid precipitates and the precursor SLN suspension is obtained. The cooling may be performed naturally by simply removing heating and exposing the emulsion to room temperature, or is preferably accelerated by subjecting the emulsion to temperatures lower to room temperature, such as by placing the vessel containing the emulsion in an ice bath.

Optionally, at step (iv) the emulsion resulting from step (iii) is subjected to a step of homogenizing by means of any method known by a person skilled in the art, for the purpose of obtaining a suspension of precursor SLNs.

"Homogenization" is understood as any process which allows reducing, by mechanical means, the size of the globules formed in the emulsion resulting from carrying out steps (i) to (iii). Examples of homogenization methods include high pressure homogenization, sonication, high-shear mixing or the application of mechanical stress or impact forces. At step (iv), the emulsion (iii) can be subjected to homogenization more than once, with the same or a different homogenization method.

The homogenization step may be omitted when the stirring of step (iii) is sufficient to reduce the emulsion globule size, and thus the suspension precursor SLN particle size to that which is desired. The stirring technique and time can affect the size of the particles. For instance, when sonication is employed as stirring technique, a stirring time of about 30 min will yield precursor SLN particles of about 100-150 nm, whereas less time will yield larger sizes, and might require a subsequent pressure homogenization step to further reduce the particle size.

In a particular embodiment, step a) further comprises a step v) of isolating the precursor SLNs from the precursor SLN suspension obtained at step iv), such as by centrifugation. In a more particular embodiment, the isolation step includes cooling the precursor SLN suspension to a temperature comprised between 4 and 8°C and subsequently isolating the precursor SLNs, such as by filtering the precursor SLNs by centrifugation.

In an embodiment, the precursor SLNs, with or without isolating, may be lyophilized. The lyophilized SLNs may be stored, and resuspended in any suspension medium at a later time for use in step c) of the process of the invention..

In an embodiment, the amount of lipid solid at room temperature comprised in the isolated precursor SLN or plurality thereof is between 10% and 90%, preferably between 40% and 80%, particularly about 65%, by weight with respect to the total weight of respectively the isolated precursor SLN or plurality thereof. These values do not include any suspension medium. In another embodiment, when in the form of a suspension, the amount of lipid solid at room temperature is comprised between 0.1% and 20%, preferably between 0.5% and 5%, by weight with respect to the total weight of the precursor SLN suspension.

In another embodiment, the amount of lipid liquid at room temperature comprised in the isolated precursor SLN or plurality thereof is between 0.1 and 30%, preferably between 1 and 20%, particularly about 7%, by weight with respect to the total weight of respectively the isolated precursor SLN or plurality thereof. These values do not include any suspension medium. In another embodiment, when in the form of a suspension, the amount of lipid liquid at room temperature is comprised between 0.01 and 5%, preferably between 0.05 and 2%, by weight with respect to the total weight of the precursor SLN suspension.

In another embodiment, the amount of cationic surfactant comprised in the isolated precursor SLN or plurality thereof is between 2.5% and 50%, preferably between 15% and 35%, particularly about 25%, by weight with respect to the total weight of respectively the isolated precursor SLN or plurality thereof. These values do not include any suspension medium. In another embodiment, when in the form of a suspension, the amount of cationic surfactant ranges between 0.05% and 10%, preferably between 0.1% and 2%, by weight with respect to the total weight of the precursor SLN suspension.

In another embodiment, the amount of non-ionic surfactant comprised in the isolated precursor SLN or plurality thereof is between 0.1 and 25%, preferably between 1 and 15%, particularly about 7%, by weight with respect to the total weight of respectively the isolated precursor SLN or plurality thereof. These values do not include any suspension medium. In another embodiment, when in the form of a suspension, the amount of non-ionic surfactant is comprised between 0.001 and 10%, preferably between 0.01 and 3%, by weight with respect to the total weight of the precursor SLN suspension.

It is understood that the sum of the specific amount chosen for each component of the precursor SLN, plurality or suspension thereof, will add up at most to 100% of weight with respect to the total weight of the precursor SLN, plurality or suspension thereof, respectively.

In another embodiment, the amount of gold added during the method of the invention is of between 10⁵ to 9*10¹⁷ number of gold particles, preferably between 10⁷ and 9*10¹⁶ number of gold particles, per mg of isolated precursor SLN. Alternatively, when the precursor SLNs are in suspension, the amount of gold added ranges between 10⁶ and 9*10¹⁹ number of gold particles, preferably between 10⁸ and 9*10¹⁷ number of gold particles, per mL of precursor SLN suspension.

The amount of nucleic acid to be added will depend in each case on the nature of the nucleic acid to be incorporated, the indication for which it is used and administration efficiency. In an embodiment, the amount of nucleic acid added is of between 0.001% and 95%, preferably between 0.1 and 60%, particularly about 7%, by weight with respect to the total weight of the isolated precursor SLN or plurality thereof. Alternatively, when the precursor SLNs are in suspension, the amount of nucleic acid added is of up to 20% by weight, preferably between 0.00001% and 20%, preferably between 0.001 and 10%, by weight with respect to the total weight of the precursor SLN suspension.

In another variant of the invention, when a polysaccharide is present, the amount of polysaccharide to be added is between 0.01 and 95%, preferably between 0.05 and 60%, particularly about 15%, by weight with respect to the total weight of the isolated precursor SLN or plurality thereof. Alternatively, when the precursor SLNs are in suspension, the amount of polysaccharide added is comprised between 0.0001 and 20%, preferably between 0.001% and 10%, by weight with respect to the total weight of the precursor SLN suspension.

In another variant of the invention, when a positively charged peptide is present, the amount of positively charged peptide to be added is comprised between 0.1% and 95%, preferably between 0.5% and 60%, particularly about 15%, by weight with respect to the total weight of the isolated precursor SLN or plurality thereof. Alternatively, when the precursor SLNs are in suspension, the amount of positively charged peptide added is comprised between 0.001 and 20%, preferably between 0.01% and 10%, by weight with respect to the total weight of the precursor SLN suspension.

The amount of precursor SLN in the precursor SLN suspension ranges from 0.5 to 100, preferably from 5 to 30, mg of precursor SLN per mL of suspension medium.

The above amounts for each of the components may all be met or only some of them may be met in which case any combination of individual amounts indicated above for each component is considered covered by the present application.

Step b) comprises preparing a solution comprising the nucleic acid and the gold, such as by methods well known to the person skilled in the art, such as by dissolving the nucleic acid and the gold in a solvent. If the SLN is to comprise a polysaccharide and/or a positively charged peptide, these are added to the solution of step b). In an embodiment, a solution of the nucleic acid and the gold is first prepared, to which - preferably after stirring - the polysaccharide and/or the positively charged peptide is then added. When both the polysaccharide and the positively charged peptide are to be added, it is preferred to add the positively charged peptide first, and then the polysaccharide is added, preferably with stirring in between the two additions. In a preferred embodiment, the solution is an aqueous solution, such as a water solution.

Step c) involves contacting the solution of step b) with the precursor SLNs obtained at step a). Preferably, the contact is performed at room temperature, such as for at least five minutes, to yield a suspension according to the present invention.

In an alternative embodiment, process Au further comprises a step d) of isolating the SLNs from the mixture obtained at step c), such as by centrifugation. In a more particular embodiment, the isolation step includes cooling the mixture to a temperature comprised between 4 and 8°C and subsequently filtering the SLNs by centrifugation.

In an embodiment, the SLNs, with or without isolating, may be lyophilized. The lyophilized SLNs may be stored, and resuspended in any suspension medium at a later time to yield a suspension according to the invention.

Excipients employed in the pharmaceutical compositions of the present invention can be added to the SLNs at any stage. Depending on the nature of the specific excipient, it will be added to the lipophilic or hydrophilic phase of the precursor SLNs during their preparation as described above, or added to the precursor SLNs after their preparation, or added to the SLNs during or after their preparation, or added to the suspension during or after its preparation.

### Process Oro

The second method of the invention differs from Process Au in that the gold is added to the precursor SLNs before they are mixed with the nucleic acid. Thus, process Oro comprises:
a) preparing a suspension of precursor gold SLN, said precursor gold SLN comprising:
   - a lipid solid at room temperature at the core;
   - a cationic surfactant;
   - a non-ionic surfactant; and
   - gold;
b) preparing a solution comprising a nucleic acid; and
c) mixing the suspension of precursor gold SLN obtained in step a) with the solution obtained in step b).

All embodiments described above for Process Au apply mutatis mutandis to Process Oro, with the exceptions that follow.

Instead of introducing the gold at step b), the gold is introduced during step a). In an embodiment, the gold is added during step (ii) of preparing an aqueous solution comprising the cationic surfactant and the non-ionic surfactant. In another embodiment, the gold is added after step (iv), in particular it is added to the precursor SLN suspension obtained at step (iv), or if step (iv) is omitted, it is added to the precursor SLN suspension obtained at step (iii). In either case, the amount of gold to be added is the same as that defined above for process Au. The precursor SLN comprising the gold (precursor gold SLN) thus obtained may be isolated, lyophilized and resuspended as described for Process Au. Similarly, the amount of the reminder of components is that defined for process Au.

The gold employed in Processes Au and Oro may be obtained by a variety of ways known to the person skilled in the art, such as those described by Kimling et al., Phys. Chem. B 2006, 110, 15700-15707; Huang et al., Chem. Eng. Sci. Volume 189, 2 November 2018, Pages 422-430; Li et al., Nanomedicine (Lond). 2015 Jan; 10(2): 299-320; or Song et al., Langmuir 2011, 27, 13854-13860. Alternatively, the gold may be obtained from commercial sources, such as from Sigma Aldrich (ID 741949 or 741965).

In an embodiment, the gold is obtained by the Turkevich method. The method comprises adding citrate solution, such as sodium citrate, to a chloroauric acid solution, at high temperatures such as at at least 80°C, and preferably at between 80°C and 100°C, e.g. at 90°C. Preferably, the chloroauric acid solution is provided as a water solution. In an embodiment, the chloroauric acid solution is heated to the immediately above stated temperature, and the sodium citrate is added once the target temperature is attained, preferably in the form of a water solution, and preferably previously heated to avoid temperature drops in the reaction mixture. Further water may be added to the reaction mixture to adjust for any loss of reaction volume. Stirring of the reaction mixture may proceed initially at the immediately above stated temperatures and subsequently at lower temperatures, such as at room temperature.

Thus, in an embodiment, when the gold is prepared by the Turkevich method, the gold may be added during the process of the invention in the form of a solution, preferably aqueous solution, comprising citrate. Alternatively, prior to adding the gold, the citrate may be removed from said solution. Methods for removing citrate are any of those known to the skilled person, such as those described in G.S. Perera et al., Journal of Colloid and Interface Science 511 (2018) 335-343.

Thus, in a related embodiment, the SLN or plurality of SLNs or suspension of the invention comprises citrate.

In any of Process Au or Oro, the ratio of gold to nucleic acid added is as follows.

In any embodiment described herein, the ratio of nucleic acid to gold nanoparticles can range from 1*10⁷ to 9*10¹² gold nanoparticles per µg of nucleic acid. A factor influencing the amount of gold nanoparticles to be added is the size of the gold nanoparticles, a higher number of nanoparticles being required for smaller sizes.

In an embodiment, the SLN is prepared according to Process Au. Preferably, in this SLN, the ratio of nucleic acid to gold nanoparticles ranges from 3.36*10⁸ to 5.36*10⁸, such as 4.36*10⁸, gold nanoparticles per µg of nucleic acid. Another embodiment is directed to this very same SLN, wherein the ratio of nucleic acid to gold nanoparticles ranges from 7.72*10⁸ to 9.72*10⁸, such as 8.72*10⁸, gold nanoparticles per µg of nucleic acid. Another embodiment is directed to this very same SLN, wherein the ratio of nucleic acid to gold nanoparticles ranges from 1.64*10⁹ to 1.84*10⁹, such as 1.74*10⁹, gold nanoparticles per µg of nucleic acid. In a different embodiment, these ratios also apply, but the SLN is prepared according to Process Oro. In any of the embodiments of this paragraph, the gold can have a size of between 15 and 25 nm, such as of 20 nm.

In an embodiment, the SLN further comprises a positively charged peptide, preferably protamine, and the SLN is prepared according to Process Au. Preferably, in this SLN, the ratio of nucleic acid to gold nanoparticles ranges from 3.36*10⁸ to 5.36*10⁸, such as 4.36*10⁸, gold nanoparticles per µg of nucleic acid. Another embodiment is directed to this very same SLN, wherein the ratio of nucleic acid to gold nanoparticles ranges from 7.72*10⁸ to 9.72*10⁸, such as 8.72*10⁸, gold nanoparticles per µg of nucleic acid. Another embodiment is directed to this very same SLN, wherein the ratio of nucleic acid to gold nanoparticles ranges from 1.64*10⁹ to 1.84*10⁹, such as 1.74*10⁹, gold nanoparticles per µg of nucleic acid. In a different embodiment, these ratios also apply, but the SLN is prepared according to Process Oro. In any of the embodiments of this paragraph, the gold can have a size of between 15 and 25 nm, such as of 20 nm.

In an embodiment, the SLN further comprises a positively charged peptide, preferably protamine, as well as a polysaccharide, preferably dextran, and the SLN is prepared according to Process Au. Preferably, in this SLN, the ratio of nucleic acid to gold nanoparticles ranges from 3.36*10⁸ to 5.36*10⁸, such as 4.36*10⁸, gold nanoparticles per µg of nucleic acid. Another embodiment is directed to this very same SLN, wherein the ratio of nucleic acid to gold nanoparticles ranges from 7.72*10⁸ to 9.72*10⁸, such as 8.72*10⁸, gold nanoparticles per µg of nucleic acid. Another embodiment is directed to this very same SLN, wherein the ratio of nucleic acid to gold nanoparticles ranges from 1.64*10⁹ to 1.84*10⁹, such as 1.74*10⁹, gold nanoparticles per µg of nucleic acid. In a different embodiment, these ratios also apply, but the SLN is prepared according to Process Oro. In any of the embodiments of this paragraph, the SLNs comprises at least two cationic surfactants, preferably DOTAP and DODAP. In any of the embodiments of this paragraph, the gold can have a size of between 15 and 25 nm, such as of 20 nm.

In an embodiment, the SLN further comprises a positively charged peptide, preferably protamine, as well as a polysaccharide, preferably hyaluronic acid, and the SLN is prepared according to Process Au. Preferably, in this SLN, the ratio of nucleic acid to gold nanoparticles ranges from 3.36*10⁸ to 5.36*10⁸, such as 4.36*10⁸, gold nanoparticles per µg of nucleic acid. Another embodiment is directed to this very same SLN, wherein the ratio of nucleic acid to gold nanoparticles ranges from 7.72*10⁸ to 9.72*10⁸, such as 8.72*10⁸, gold nanoparticles per µg of nucleic acid. Another embodiment is directed to this very same SLN, wherein the ratio of nucleic acid to gold nanoparticles ranges from 1.64*10⁹ to 1.84*10⁹, such as 1.74*10⁹, gold nanoparticles per µg of nucleic acid. In a different embodiment, these ratios also apply, but the SLN is prepared according to Process Oro. In any of the embodiments of this paragraph, the gold can have a size of between 15 and 25 nm, such as of 20 nm.

In an embodiment, the SLN is prepared according to Process Au. Preferably, in this SLN, the ratio of nucleic acid to gold nanoparticles ranges from 1.4*10¹⁰ to 0.1*10¹⁰, such as 0.73*10¹⁰, gold nanoparticles per µg of nucleic acid. Another embodiment is directed to this very same SLN, wherein the ratio of nucleic acid to gold nanoparticles ranges from 2.67*10¹⁰ to 4.67*10¹⁰, such as 3.67*10¹⁰, gold nanoparticles per µg of nucleic acid. Another embodiment is directed to this very same SLN, wherein the ratio of nucleic acid to gold nanoparticles ranges from 6.33*10¹⁰ to 8.33*10¹⁰, such as 7.33*10¹⁰, gold nanoparticles per µg of nucleic acid. In a different embodiment, these ratios also apply, but the SLN is prepared according to Process Oro. In any of the embodiments of this paragraph, the gold can have a size of between 2 and 8 nm, such as of 5 nm.

In an embodiment, the SLN further comprises a positively charged peptide, preferably protamine, and the SLN is prepared according to Process Au. Preferably, in this SLN, the ratio of nucleic acid to gold nanoparticles ranges from 1.73*10¹⁰ to 0.1*10¹⁰, such as 0.73*10¹⁰, gold nanoparticles per µg of nucleic acid. Another embodiment is directed to this very same SLN, wherein the ratio of nucleic acid to gold nanoparticles ranges from 2.67*10¹⁰ to 4.67*10¹⁰, such as 3.67*10¹⁰, gold nanoparticles per µg of nucleic acid. Another embodiment is directed to this very same SLN, wherein the ratio of nucleic acid to gold nanoparticles ranges from 6.33*10¹⁰ to 8.33*10¹⁰, such as 7.33*10¹⁰, gold nanoparticles per µg of nucleic acid. In a different embodiment, these ratios also apply, but the SLN is prepared according to Process Oro. In any of the embodiments of this paragraph, the gold can have a size of between 2 and 8 nm, such as of 5 nm.

In an embodiment, the SLN further comprises a positively charged peptide, preferably protamine, as well as a polysaccharide, preferably dextran, and the SLN is prepared according to Process Au. Preferably, in this SLN, the ratio of nucleic acid to gold nanoparticles ranges from 1.73*10¹⁰ to 0.1*10¹⁰, such as 0.73*10¹⁰, gold nanoparticles per µg of nucleic acid. Another embodiment is directed to this very same SLN, wherein the ratio of nucleic acid to gold nanoparticles ranges from 2.67*10¹⁰ to 4.67*10¹⁰, such as 3.67*10¹⁰, gold nanoparticles per µg of nucleic acid. Another embodiment is directed to this very same SLN, wherein the ratio of nucleic acid to gold nanoparticles ranges from 6.33*10¹⁰ to 8.33*10¹⁰, such as 7.33*10¹⁰, gold nanoparticles per µg of nucleic acid. In a different embodiment, these ratios also apply, but the SLN is prepared according to Process Oro. In any of the embodiments of this paragraph, the SLNs comprises at least two cationic surfactants, preferably DOTAP and DODAP. In any of the embodiments of this paragraph, the gold can have a size of between 2 and 8 nm, such as of 5 nm.

In an embodiment, the SLN further comprises a positively charged peptide, preferably protamine, as well as a polysaccharide, preferably hyaluronic acid, and the SLN is prepared according to Process Au. Preferably, in this SLN, the ratio of nucleic acid to gold nanoparticles ranges from 1.73*10¹⁰ to 0.1*10¹⁰, such as 0.73*10¹⁰, gold nanoparticles per µg of nucleic acid. Another embodiment is directed to this very same SLN, wherein the ratio of nucleic acid to gold nanoparticles ranges from 2.67*10¹⁰ to 4.67*10¹⁰, such as 3.67*10¹⁰, gold nanoparticles per µg of nucleic acid. Another embodiment is directed to this very same SLN, wherein the ratio of nucleic acid to gold nanoparticles ranges from 6.33*10¹⁰ to 8.33*10¹⁰, such as 7.33*10¹⁰, gold nanoparticles per µg of nucleic acid. In a different embodiment, these ratios also apply, but the SLN is prepared according to Process Oro. In any of the embodiments of this paragraph, the gold can have a size of between 2 and 8 nm, such as of 5 nm.

In any of the above embodiments, the weight/weight ratio of lipid solid at room temperature added during step a) to nucleic acid added during step b) is from 1:1 to 10:1, preferably from 3:1 to 7:1, more preferably it is 5:1.

The suspension of the invention may be employed without removing gold, nucleic acid, polysaccharide or positively charged peptide which is not in the hydrophilic phase of or adsorbed onto the surface of the SLNs of the invention. These components are herein referred to as "free" components and can be by-products of some preparation methods of the system or suspension of the invention.

Thus, in a related embodiment, the suspension of the invention further comprises gold which is found in the suspension medium and is not found in the hydrophilic phase of or adsorbed onto the surface of the SLNs of the invention. Therefore, in an embodiment, at most 80%, preferably at most 50%, even more preferably at most 20%, by weight of the gold in the suspension of the invention with respect to the total weight of the gold in said suspension is free gold.

Similarly, in a related embodiment, the suspension of the invention further comprises nucleic acid which is found in the suspension medium and is not found in the hydrophilic phase of or adsorbed onto the surface of the SLNs of the invention. Therefore, in an embodiment, at most 80%, preferably at most 50%, even more preferably at most 20%, by weight of the nucleic acid in the suspension of the invention with respect to the total weight of nucleic acid in said suspension is free nucleic acid. The weights of free nucleic acid in the case of single and double stranded nucleic acid may differ due to their different molecular architecture and thus also different interaction with the SLNs of the invention. Thus, in an embodiment, the amount of free nucleic acid is selected from the above list independently for each of these nucleic acids.

Similarly, in a related embodiment, the suspension of the invention further comprises polysaccharide which is found in the suspension medium and is not found in the hydrophilic phase of or adsorbed onto the surface of the SLNs of the invention. Therefore, in an embodiment, at most 80%, preferably at most 50%, even more preferably at most 20%, by weight of the polysaccharide in the suspension of the invention with respect to the total weight of the polysaccharide in said suspension is free polysaccharide.

Similarly, in a related embodiment, the suspension of the invention further comprises positively charged peptide which is found in the suspension medium and is not found in the hydrophilic phase of or adsorbed onto the surface of the SLNs of the invention. Therefore, in an embodiment, at most 80%, preferably at most 50%, even more preferably at most 20%, by weight of the positively charged peptide in the suspension of the invention with respect to the total weight of the positively charged peptide in said suspension is free positively charged peptide.

Similarly, SLNs which have no gold in their hydrophilic phase nor adsorbed onto the surface of the SLN may also be comprised in the suspension of the invention. Therefore, in an embodiment, at most 80%, preferably at most 50%, even more preferably at most 20%, by weight of all of the SLNs in the suspension are SLNs which have no gold in their hydrophilic phase nor adsorbed onto the surface of the SLN.

In another important non-claimed aspect, the disclosure is directed to an SLN, or a plurality thereof, or a suspension thereof, obtained by any of the processes described herein in any of the above described embodiments.

For instance, the suspension of the disclosure is one obtained by preparing a precursor SLN suspension as described anywhere herein, such as in the amounts described herein, and adding gold, nucleic acid and optionally the remainder of components of the SLN of the invention, in the amounts described herein.

In another non-claimed aspect, the disclosure is directed to a precursor gold SLN as described above.

Another object of the present invention is a pharmaceutical composition comprising the SLN or the plurality of SLNs of the invention, or the suspension of the invention, and a pharmaceutically acceptable excipient.

The term "excipient" refers to a vehicle, diluent or adjuvant that is administered with the SLNs in order to achieve or facilitate the desired pharmacological action. Suitable pharmaceutical excipients are described in "Remington's Pharmaceutical Sciences" by E.W. Martin, 1995, and include solubilizers; anti-flocculating agents; stabilizers, especially those which are steric or ionic or surfactant in nature; viscosity modulators; pH controlling agents such as for example buffer agents which prevent the pH of the composition from dropping to values less than 5; antioxidant agents which inhibit the oxidation of the lipid component; as well as preservatives which prevent important structural changes in the formulation.

Depending on their function and hydrophilicity, these excipients can be present in either of the phases making up the SLNs or adsorbed thereon, or in the suspension medium where the SLNs are dispersed.

The pharmaceutical composition according to the present invention may be in any form suitable for the application to humans and/or animals, such as topical or systemic application, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes. A preferred route of administration is the ocular route, e.g. by subretinal injection, intravitreal injection or subconjunctival injection, or by topical administration to the eye. A preferred route of ocular administration is the topical administration to the eye, in particular to the cornea. Another particular route of administration is the intravenous route. Another particular route of administration is the intramuscular route. Another particular route of administration is the oral route.

Another aspect of the invention refers to the SLN or the plurality of SLNs or the suspension or the pharmaceutical composition of the invention, for use as a medicament, preferably for use in gene therapy.

Similarly, the invention relates to the use of the SLN or the plurality of SLNs or the suspension or the pharmaceutical composition of the invention, in the manufacture of a medicament for gene therapy.

Similarly, the disclosure relates to a non-claimed method of gene therapy, the method comprising administering to a patient in need of such therapy a therapeutically effective amount of the SLN or the plurality of SLNs or the suspension or the pharmaceutical composition of the invention.

In the context of the present invention, the term "gene therapy" refers to treatment of a subject which involves insertion of a nucleic acid into the cells of said subject for the purpose of preventing or treating disease. The introduction of the nucleic acid into the host's cells typically achieves expression of said nucleic acid to yield an expression product which effects the prevention or treatment of the disease, such as functional protein that compensates for the absence, underexpression or functional deficiency of the protein in the subject. Alternatively, gene therapy may involve the silencing or editing of genes the expression or overexpression of which causes disease.

The term "treatment" and derived terms in the context of this document refers to the improvement or elimination of the disease or of one or more symptoms associated with the disease. The term "prevention" and derived terms refers to eliminating or reducing the risk of the disease appearing or developing.

When used for preventing or treating disease, the SLNs of the present invention are employed in therapeutically effective amounts. The physician will determine the dosage of SLNs which is most suitable on a case to case basis, bearing into account the specific form of administration, the specific profile of the patient under treatment, and the specific type of disease to treat.

The present inventors have surprisingly found that the SLNs of the present invention are capable of transfecting and enabling nucleic acid expression in a variety of cells of different nature.

Thus, in an embodiment, the disease to be treated is selected from an ocular disease and a cardiac disease.

In a particular embodiment, the disease to be treated is an ocular disease. Examples of such diseases are glaucoma; macular degeneration: retinopathy, including diabetic retinopathy and ischemic retinopathy; corneal infections or inflammations, such as herpes simplex; Leber's congenital amaurosis; corneal surface opacity; retinitis pigmentosa; albinism; choroideremia; X-linked juvenile retinoschisis; Stargardt's disease; retinoblastoma; granular corneal dystrophy; Fuchs' corneal dystrophy; gelatinous drop-like corneal dystrophy; corneal transplant rejection; and corneal surface opacity associated with mucopolysaccharidosis. A preferred disease is X-linked juvenile retinoschisis.

In a particular embodiment, the disease to be treated is a cardiac disease. Examples of such diseases are cardiac hypertrophy, such as ventricular hypertrophy; cardiac hypertrophy-associated or autophagic disorders, such as pulmonal vein stenosis, atrial or ventricular septum defect, hypertrophic non-obstructive or obstructive cardiomyopathy, or Fabry disease; and/or cardiac dysfunction such as contractile dysfunction, cardiac decompensation or heart failure, ventricular remodeling after myocardial infarction, or myocarditis.

In a particular embodiment, the disease to be treated is a lysosomal storage disease. Lysosomal storage diseases are a class of approximately 50 different human metabolic diseases caused by a deficiency for specific lysosomal proteins that results in the accumulation of various substances within the endosomal/lysosomal compartments. Examples of such diseases are Pompe Disease, Fabry Disease, and Gaucher Disease, MPS I, MPSII, MPS VII, Tay Sachs, Sandhoff, α-mannosidosis, or Wohlman disease. A preferred disease is Fabry disease.

The present invention is also directed to the cosmetic use, more specifically the cosmetic genetic therapy use, of the SLN or the plurality of SLNs or the suspension of the invention. Examples of such uses are the treatment of oily skin, acne, skin dryness, undesired skin tone or ageing or the effects thereof such as wrinkles or the lack of skin firmness. As explained for the therapeutic uses above, cosmetic gene therapy involves genes which relate to the above cosmetic conditions.

Some illustrative examples which clearly show the features and advantages of the invention are described below, nevertheless, they must not be interpreted as limiting the object of the invention as defined in the claims.

### Examples

The SLNs of the present invention were characterized by the following methods:
1. Microscope images
   Gold particles and SLNs were observed by Transmission Electron Microscopy (TEM). Ten µl of the sample was adhered onto glow discharged carbon coated grids for 60 s. After removing the remaining liquid, via blotting on filter paper, the staining was carried out with 2% uranyl acetate for 60 s. Samples were visualized using a Philips EM208S TEM and digital images were acquired on an Olympus SIS purple digital camera.
2. Particle size and surface charge
   The particle size and PDI of gold particles and SLNs was measured by means of Dynamic Light Scattering (DLS), more specifically Photon Correlation Spectroscopy (PCS). The surface charge was measured by means of laser doppler velocimetry. Both parameters ere measured by Zetasizer Nano ZS.
3. Transfection & Expression studies
   Nucleic acid transfection and expression studies were carried out in ARPE-19 (human retinal pigment epithelial), HEK-293 (Human embryonic kidney), HL-1 (adult rat cardiomyocyte) and/or (Immortalized Fabry Endothelial **cell line**-1) cell lines. ARPE-19 cells were kept in culture in Dulbecco's Modified Eagle Medium:Nutrient Mixture F-12 (DMEM / F-12) with 10% fetal bovine serum, 1% penicillin and streptomycin antibiotic. HEK293 cells were kept in culture in Eagle's Minimum Essential Medium (EMEM) with 10% fetal bovine serum, 1% penicillin and streptomycin antibiotic. HL-1 cells were kept in culture in Claycomb Medium with 10% fetal bovine serum, 1% penicillin and streptomycin antibiotic, 0.1 mM norepinephrine and 2mM L-Glutamine. IMFE-1 cells were kept in containing EBM^{™}-2 Basal Medium and EGM^{™}-2 SingleQuots^{™} Supplements. The cell cultures were kept at 37°C in a 5% CO2 air atmosphere, changing the medium every 2 or 3 days.

For ARPE-19 cells the transfection studies were conducted in 12-well plates with densities of 60,000 cells per well. For HEK293 cells the transfection studies were conducted in 24-well plates with densities of 100,000 cells per well. For HL-1 cells transfection studies were carried out in 24-well plates with densities of 150,000 cells per well. For IMFE cells the transfection studies were conducted in 24-well plates with 30,000 cells per well. The cultures were left to incubate until reaching 80-90% confluence. When reaching 80-90% confluence part of the medium was removed, leaving the necessary volume for covering the entire well, then adding the nanoparticle system to be studied. They were incubated for 4 hours and more volume of the fresh medium was then added. The amount of vectors added to each well was equivalent to 2.5 µg of DNA or mRNA.

### Example 1 Preparation of gold nanoparticles

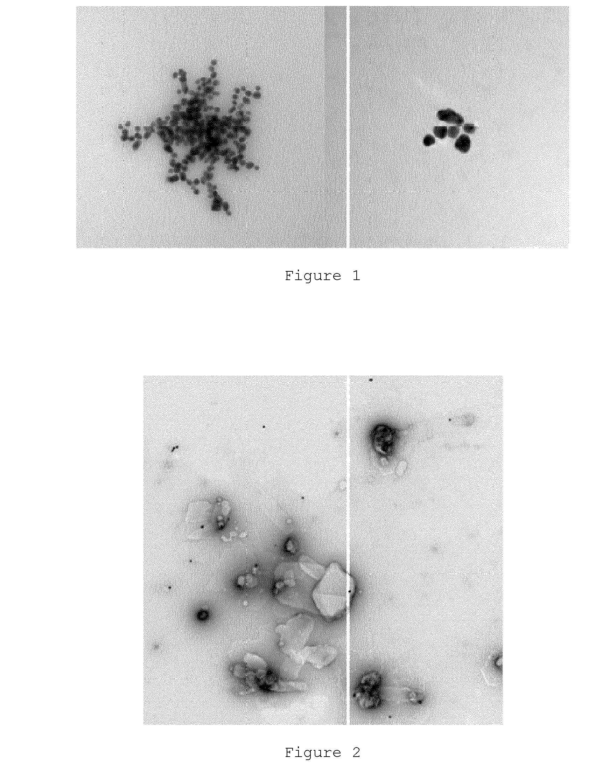

Gold nanoparticles (AuNP) were either obtained from Sigma Aldrich (Ref. 741965) or prepared by the Turkevich method. 10 mL of 1 mM tetrachlorouric acid (HAuCl₄) solution were heated to 90°C and 1 mL of 38.8 mM sodium citrate (Na₃C₆H₅O₇) solution was then added under continuous stirring. After 2 minutes from the citrate addition, 3 mL of distilled water were added to maintain the reaction volume. After 5 minutes from the citrate addition (intense red color) 1.5 mL of distilled water were added, heating was stopped and the reaction mixture was left stirring. After 8 minutes from the citrate addition, stirring was ceased and the reaction mixture was allowed to cool down. TEM images of the gold nanoparticles are shown in Figure 1.

### Example 2 (comparative). Preparation of SLNs without gold, positively charged peptide or polysaccharide (SLN)

A 5% precirol solution in dichloromethane (2 mL) is prepared. In addition an aqueous solution (10 mL) of 0.4% DOTAP and 0.1% Tween 80 is prepared. The aqueous phase is added to the oily phase, subjecting the mixture to a rigorous stirring until obtaining an emulsion. Then the organic solvent is evaporated, keeping the emulsion under mechanical stirring for at least 5 minutes, subsequently subjecting it to vacuum for at least 5 minutes. The lipid thus precipitates, a SLN suspension being obtained. After cooling to a temperature between 4 and 8°C, the SLNs are filtered by centrifugation and resuspended in purified water.

0,2 µg/µL of nucleic acid (either pcDNA3-EGFP or CleanCap^{®} EGFP mRNA)solution in distilled water is prepared. The SLN suspension is then contacted with the solution containing the nucleic acid (either mRNA or DNA) at a 5:1 ratio (expressed as the weight/weight ratio between DOTAP and nucleic acid) and is kept under stirring for 20 minutes at room temperature. The resulting mixture is tested as is.

### Example 3 (comparative). Preparation of SLNs without gold or polysaccharide but with positively charged peptide (SLN-P).

The SLNs were prepared as described in Example 2 only that protamine was added to the solution containing the nucleic acid and the mixture was stirred for 5 minutes before contact with SLN suspension.

### Example 4 (comparative). Preparation of SLNs without gold but with positively charged peptide and polysaccharide (dextran) (SLN-P-DX)

The SLNs were prepared as described in Example 2 only that protamine and dextran were added to the solution containing the nucleic acid. The mixture of protamine and nucleic acid was stirred for 5 minutes before contact with dextran. The mixture of protamine, nucleic acid and dextran was stirred for 15 minutes before contact with SLN suspension.

### Example 5 (comparative). Preparation of SLNs without gold but with positively charged peptide and polysaccharide (hyaluronic acid) (SLN-P-HA)

The SLNs were prepared as described in Example 2 only that protamine and hyaluronic acid were added to the solution containing the nucleic acid. The mixture of protamine and nucleic acid was stirred for 5 minutes before contact with hyaluronic acid. The mixture of protamine, nucleic acid and hyaluronic acid was stirred for 15 minutes before contact with SLN suspension.

### Example 6. Preparation of SLNs with gold and without positively charged peptide or polysaccharide by means of Process Au (SLN Au)

The SLNs were prepared as described in Example 2 only that the gold nanoparticles of Example 1 were added to the solution containing the nucleic acid and the mixture was stirred for 15 minutes before contact with SLN suspension.

### Example 7. Preparation of SLNs with gold and without polysaccharide but with positively charged peptide by means of Process Au (SLN Au-P)

The SLNs were prepared as described in Example 2 only that the gold nanoparticles of Example 1 as well as protamine were added to the solution containing the nucleic acid. The mixture of gold nanoparticles and nucleic acid was stirred for 15 minutes before contact with protamine. The mixture of gold nanoparticles, nucleic acid and protamine was stirred for 5 minutes before contact with SLN suspension.

### Example 8. Preparation of SLNs with gold, positively charged peptide and polysaccharide (dextran) by means of Process Au (SLN Au-P-DX)

The SLNs were prepared as described in Example 2 only that the gold nanoparticles of Example 1 as well as protamine and dextran were added to the solution containing the nucleic acid. The mixture of gold nanoparticles and nucleic acid was stirred for 15 minutes before contact with protamine. The mixture of gold nanoparticles, nucleic acid and protamine was stirred for 5 minutes before contact with dextran. The mixture of gold nanoparticles, nucleic acid, protamine and dextran was stirred for 15 minutes before contact with SLN suspension.

### Example 9. Preparation of SLNs with gold, positively charged peptide and polysaccharide (hyaluronic acid) by means of Process Au (SLN Au-P-HA)

The SLNs were prepared as described in Example 2 only that the gold nanoparticles of Example 1 as well as protamine and hyaluronic acid were added to the solution containing the nucleic acid. The mixture of gold nanoparticles and nucleic acid was stirred for 15 minutes before contact with protamine. The mixture of gold nanoparticles, nucleic acid and protamine was stirred for 5 minutes before contact with hyaluronic acid. The mixture of gold nanoparticles, nucleic acid, protamine and hyaluronic acid was stirred for 15 minutes before contact with SLN suspension. A TEM image of the obtained SLN system is shown at Figure 2.

### Example 10. Preparation of SLNs with gold and without positively charged peptide or polysaccharide by means of Process Oro (SLN Oro)

The SLNs were prepared as described in Example 2 only that the gold nanoparticles of Example 1 were added to the aqueous phase during the preparation of the precursor gold SLN.

### Example 11. Preparation of SLNs with gold and without polysaccharide but with positively charged peptide by means of Process Oro (SLN Oro-P)

The SLNs were prepared as described in Example 2 only that the gold nanoparticles of Example 1 were added to the aqueous phase during the preparation of precursor gold SLN, and protamine was added to the solution containing the nucleic acid; the mixture of protamine and nucleic acid was stirred for 5 minutes before contact with precursor gold SLN.

### Example 12. Preparation of SLNs with gold, positively charged peptide and polysaccharide (dextran) by means of Process Oro (SLN Oro-P-DX)

The SLNs were prepared as described in Example 2 only that the gold nanoparticles of Example 1 were added to the aqueous phase during the preparation of precursor gold SLN, and protamine and dextran were added to the solution containing the nucleic acid. The mixture of protamine and nucleic acid was stirred for 5 minutes before contact with dextran. The mixture of protamine, nucleic acid and dextran was stirred for 15 minutes before contact with precursor gold SLN.

### Example 13. Preparation of SLNs with gold, positively charged peptide and polysaccharide (hyaluronic acid) by means of Process Oro (SLN Oro-P-HA)

The SLNs were prepared as described in Example 2 only that the gold nanoparticles of Example 1 were added to the aqueous phase during the preparation of precursor gold SLN, and protamine and dextran were added to the solution containing the nucleic acid. The mixture of protamine and nucleic acid was stirred for 5 minutes before contact with hyaluronic acid. The mixture of protamine, nucleic acid and hyaluronic acid was stirred for 15 minutes before contact with precursor gold SLN.

### Example 14. Physical properties of SLNs

The size, dispersity and superficial charge of SLNs comprising mRNA or plasmid DNA as nucleic acid were measured. Examples are shown below. SLN on its own denotes a solid lipid nanoparticle comprising no gold. Extensions _Au or _Oro denote an SLN according to the present invention, prepared according to Process Au or Process Oro, respectively.

| | Size / nm | PDI | Zeta potential / mV |
|---|---|---|---|
| Gold | 27.1 ± 2.1 | 0.37 ± 0.03 | -45.1 ± 1.4 |
| SLN | 268.60 ± 9.97 | 0.41 ± 0.02 | 36.07 ± 0.80 |
| SLN_Oro | 267.23 ± 6.60 | 0.42 ± 0.03 | 36.75 ± 0.92 |
| SLN-P | 167.00 ± 3.94 | 0.22 ± 0.02 | 38.02 ± 4.29 |
| SLN_Au-P | 164.53 ± 0.06 | 0.23 ± 0.01 | 37.35 ± 1.13 |
| SLN-P-HA | 602.93 ± 37.48 | 0.89 ± 0.1 | 21.28 ± 0.88 |
| SLN_Au-P-HA | 208.97 ± 3.62 | 0.34 ± 0.01 | 29.35 ± 0.39 |
| SLN_P-DX* | 164.50 ± 4.04 | 0.31 ± 0.03 | 48.17 ± 5.47 |
| SLN_Au-P-DX* | 154.33 ± 4.71 | 0.30 ± 0.04 | 47.77 ± 1.90 |
| SLN_Oro-P-DX* | 151.33 ± 1.70 | 0.28 ± 0.03 | 43.75 ± 1.74 |

| | | | |
|---|---|---|---|
| **test was carried out with commercial 5 nm gold nanoparticles and DNA as nucleic acid.* | | | |

As can be observed, despite the fact of adding a further component to the SLN system, the incorporation of the gold resulted either in no size/PDI increase or even yielded SLNs with lower size/PDI. This effect was particularly marked in the SLN comprising the GAG polysaccharide.

### Example 15. Transfection power of SLNs

The percentage of transfected cells achieved in different cell lines with different SLN systems was measured. Representative examples are shown below.

| | Cell line | Nucleic Acid | % Transfected cells |
|---|---|---|---|
| SLN | ARPE-19 | mRNA | 24.01 ± 0.82 |
| SLN_Au | ARPE-19 | mRNA | 54.65 ± 1.08 |
| SLN-P | ARPE-19 | mRNA | 66.25 ± 1.26 |
| SLN_Au-P | ARPE-19 | mRNA | 84.85 ± 1.40 |
| SLN-P-HA | ARPE-19 | mRNA | 79.04 ± 5.06 |
| SLN_Au-P-HA | ARPE-19 | mRNA | 83.34 ± 0.01 |
| SLN-P-DX* | ARPE-19 | DNA | 7.75 ±0 .76 |
| SLN_Au-P-DX* | ARPE-19 | DNA | 9.48 ± 0.74 |
| SLN | HEK-293 | DNA | 21.69 ± 0.11 |
| SLN_Oro | HEK-293 | DNA | 26.21 ± 0.59 |
| SLN-P-DX | HEK-293 | DNA | 1.53 ± 1.84 |
| SLN_Oro-P-DX | HEK-293 | DNA | 5.71 ± 0.59 |
| SLN-P-DX | HEK-293 | mRNA | 4.52 ± 0.04 |
| SLN_Oro-P-DX | HEK-293 | mRNA | 48.51 ± 0.78 |
| SLN | HL-1 | mRNA | 6.69 ± 1.49 |
| SLN_Au | HL-1 | mRNA | 15.35 ± 0.85 |
| SLN-P-DX* | IMFE-1 | DNA | 29.29 ± 1.72 |
| SLN_Oro-P-DX | IMFE-1 | DNA | 37.05 ± 2.26 |

| | | | |
|---|---|---|---|
| **test was carried out with commercial 5 nm gold nanoparticles* | | | |

As can be observed, the incorporation of gold into the SLNs yielded increased transfection levels. Increased transfection was observed both for mRNA and DNA as well as in very distinct cell lines.

### Example 16. Expression power of SLNs

The intensity of fluorescence emitted by cells after transfection therein of GFP-encoding-nucleic acid was measured. Representative examples are shown below.

| | Cell line | Nucleic Acid | Fluorescence intensity / RFU |
|---|---|---|---|
| SLN | ARPE-19 | DNA | 15823.35 ± 2133 |
| SLN_Oro | ARPE-19 | DNA | 274320.35 ± 1800 |
| SLN | ARPE-19 | DNA | 15823.35 ± 2133 |
| SLN_Au | ARPE-19 | DNA | 260473.05 ± 14960 |
| SLN-P-DX* | ARPE-19 | DNA | 195092.05 ± 32068 |
| SLN_Au-P-DX* | ARPE-19 | DNA | 283810.20 ± 66701 |
| SLNOro-P-DX* | ARPE-19 | DNA | 230255. ± 6449 |
| SLN-P | HEK-293 | mRNA | 351519.20 ± 12858 |
| SLN_Au-P | HEK-293 | mRNA | 419097.10 ± 20998 |
| SLN-P-HA | ARPE-19 | mRNA | 202270.10 ± 111 |
| SLN_Oro-P-HA | ARPE-19 | mRNA | 453336.33 ± 19 |
| SLN-P-HA | ARPE-19 | mRNA | 202270.10 ± 111 |
| SLN_Au-P-HA | ARPE-19 | mRNA | 267546.23 ± 21 |
| SLN-P-DX | HEK-293 | DNA | 249593.90 ± 10445 |
| SLN_Oro-P-DX | HEK-293 | DNA | 482162.45 ± 18800 |
| SLN-P-DX | ARPE-19 | mRNA | 125533.45 ± 893 |
| SLN_Oro-P-DX | ARPE-19 | mRNA | 495108.55 ± 85 |
| SLN | HL-1 | mRNA | 9543.77 ± 1136 |
| SLN_Au | HL-1 | mRNA | 63564.67 ± 2990 |
| SLN-P-DX | IMFE-1 | mRNA | 109838.00 ± 7745 |
| SLN_Au-P-DX | IMFE-1 | mRNA | 195672.63 ± 23612 |
| SLN-P-DX* | IMFE-1 | DNA | 470962.17 ± 13412 |
| SLN_Au-P-DX* | IMFE-1 | DNA | 555295.83 ± 31727 |
| SLN_Oro-P-DX | IMFE-1 | DNA | 535023.03 ± 34748 |

| | | | |
|---|---|---|---|
| **test was carried out with commercial 5 nm gold nanoparticles* | | | |

As can be observed, the incorporation of gold into the SLNs yielded increased nucleic acid expression levels. Increased expression was observed both for mRNA and DNA as well as in very distinct cell lines.

### Example 17. SLNs prepared by hot-melt emulsification and transfection/expression power

100 mg precirol were heated to 75-85 °C. In addition, an aqueous solution (10 mL H₂O) of 40 mg DOTAP and 10 mg Tween 80 is prepared and heated to 75-85 °C. The aqueous phase is added to the oily phase, the mixture is subjected to sonication during 30 minutes at a temperature of 75-85 °C, and the resulting mixture is allowed to cool down in ice to precipitate the lipid and obtain an SLN suspension, to which the gold nanoparticles of Example 1 are added.

CleanCap^{®} EGFP mRNA solution in distilled water is prepared. Firstly protamine, and then dextran is added to the solution, which is then stirred for 15 min. The precursor gold SLN suspension is then contacted with the solution containing the nucleic acid, protamine and dextran at a 5:1 ratio (expressed as the weight/weight ratio between DOTAP and nucleic acid) and the mixture is kept under stirring for 20 minutes at room temperature. The resulting formulation is tested as is in ARPE-19 cell line.

The percentage of transfected cells was measured and compared with that of an SLN formulation not comprising the gold:

| | Cell line | Nucleic Acid | % Transfected cells |
|---|---|---|---|
| SLN-P-DX | ARPE-19 | mRNA | 71.46 ± 1.52 |
| SLN_Oro-P-DX | ARPE-19 | mRNA | 75.27 ± 1.08 |

The intensity of fluorescence emitted by cells after transfection therein of the GFP-encoding-nucleic acid was also measured:

| | Cell line | Nucleic Acid | Fluorescence intensity / RFU |
|---|---|---|---|
| SLN-P-DX | ARPE-19 | mRNA | 263443 ± 10700 |
| SLN_Oro-P-DX | ARPE-19 | mRNA | 365073 ± 23981 |

### Example 18. SLNs prepared by hot-melt emulsification and transfection/expression power

The SLNs were prepared as described in Example 17 only that 30 mg of CTAB were employed as the cationic surfactant and 20 mg Poloxamer 338 were used as the non-ionic surfactant. Additionally, a second set of SLNs are prepared with the same components, but adding the gold to the nucleic acid, protamine and dextran prior to contact with the precursor SLNs (Process Au). The percentage of transfected cells was measured and compared with that of an SLN formulation not comprising the gold:

| | Cell line | Nucleic Acid | % Transfected cells |
|---|---|---|---|
| SLN-P-DX | ARPE-19 | mRNA | 5.91 ± 1.25 |
| SLN_Oro-P-DX | ARPE-19 | mRNA | 11.39 ± 3.86 |
| SLN_Au-P-DX | ARPE-19 | mRNA | 13.12 ± 2.46 |

The intensity of fluorescence emitted by cells after transfection therein of the GFP-encoding-nucleic acid was also measured:

| | Cell line | Nucleic Acid | Fluorescence intensity / RFU |
|---|---|---|---|
| SLN-P-DX | ARPE-19 | mRNA | 3013.80 ± 228.78 |
| SLN_Oro-P-DX | ARPE-19 | mRNA | 3540.03 ± 428.27 |
| SLN_Au-P-DX | ARPE-19 | mRNA | 3320.27 ± 527.12 |

### Example 19. Preparation of SLNs with gold, positively charged peptide and polysaccharide (dextran) by means of Process Au (SLN Au-P-DX) and transfection/expression power

The SLNs were prepared as described in Example 8 only that half the amount of DOTAP was replaced with DODAP.

The percentage of transfected cells was measured and compared with that of an SLN formulation not comprising the gold:

| | Cell line | Nucleic Acid | % Transfected cells |
|---|---|---|---|
| SLN-P-DX | HEK-293 | mRNA | 42.13 ± 0.76 |
| SLN_Au-P-DX | HEK-293 | mRNA | 56.40 ± 2.31 |

The intensity of fluorescence emitted by cells after transfection therein of the GFP-encoding-nucleic acid was also measured:

| | Cell line | Nucleic Acid | Fluorescence intensity / RFU |
|---|---|---|---|
| SLN-P-DX | ARPE-19 | mRNA | 311369 ± 12034 |
| SLN_Au-P-DX | ARPE-19 | mRNA | 395461 ± 117 |

### Example 20. Preparation of SLNs with gold, positively charged peptide and polysaccharide (dextran) by means of Process Oro (SLN Oro-P-DX) and transfection/expression power

The SLNs were prepared as described in Example 12 only that half the amount of Tween 80 was replaced with Poloxamer 338.

The percentage of transfected cells was measured and compared with that of an SLN formulation not comprising the gold:

| | Cell line | Nucleic Acid | % Transfected cells |
|---|---|---|---|
| SLN-P-DX | ARPE-19 | mRNA | 82.60 ± 2.04 |
| SLN_Oro-P-DX | ARPE-19 | mRNA | 88.03 ± 0.59 |

The intensity of fluorescence emitted by cells after transfection therein of the GFP-encoding-nucleic acid was also measured:

| | Cell line | Nucleic Acid | Fluorescence intensity / RFU |
|---|---|---|---|
| SLN-P-DX | ARPE-19 | mRNA | 660812 ± 42769 |
| SLN_Oro-P-DX | ARPE-19 | mRNA | 758814 ± 12685 |

### Example 21. Preparation of SLNs with gold, positively charged peptide and polysaccharide (dextran) by means of Process Au (SLN Au-P-DX) or Process Oro (SLN Oro-P-DX) and transfection/expression power

The SLNs were prepared as described in Example 8 or 12 only that glyceryl monostearate was employed instead of precirol.

The percentage of transfected cells was measured and compared with that of an SLN formulation not comprising the gold:

| | Cell line | Nucleic Acid | % Transfected cells |
|---|---|---|---|
| SLN-P-DX | ARPE-19 | mRNA | 66.16 ± 1.62 |
| SLN Au-P-DX | ARPE-19 | mRNA | 84.35 ± 1.84 |
| SLN_Oro-P-DX | ARPE-19 | mRNA | 74.96 ± 0.90 |

The intensity of fluorescence emitted by cells after transfection therein of the GFP-encoding-nucleic acid was also measured:

| | Cell line | Nucleic Acid | Fluorescence intensity / RFU |
|---|---|---|---|
| SLN-P-DX | ARPE-19 | mRNA | 142407 ± 8993 |
| SLN Au-P-DX | ARPE-19 | mRNA | 239483 ± 7875 |
| SLN_Oro-P-DX | ARPE-19 | mRNA | 157815 ± 3448 |

### Example 22. Preparation of lyophilized SLNs with gold, positively charged peptide and polysaccharide (dextran) by means of Process Oro (SLN Oro-P-DX) and transfection power

The SLNs were prepared as described in Example 12, only that the precursor gold SLNs were isolated, lyophilized, stored in lyophilized state during one month, and reconstituted into a suspension, before contacting with the solution comprising the protamine, dextran and nucleic acid.

The percentage of transfected cells achieved with SLNs according to Example 12, with an equivalent formulation not comprising gold, and with the SLNs according to Example 12 with intermediate lyophilization as described above, was assessed:

| | Cell line | Nucleic Acid | % Transfected cells |
|---|---|---|---|
| SLN-P-DX | ARPE-19 | mRNA | 59.39 ± 1.55 |
| SLN Oro-P-DX | ARPE-19 | mRNA | 74.03 ± 2.41 |
| SLN_Oro-P-DX (with lyo.) | ARPE-19 | mRNA | 71.64 ± 1.46 |

### Example 23. In vivo assay

To evaluate the capacity of the SLNs of the invention to transfect the cornea, eye drops containing either SLN-P-HA or SLN_Au-P-HA with CleanCap^{™} EGFP mRNA (TriLink BioTechnologies) as nucleic acid were administered topically on the ocular surface of C57BL/6 mice. This mRNA encodes the reporter GFP, which is an intracellular protein. The *in vivo* study was approved by the Animal Experimentation Ethics Committee of the University of the Basque Country UPV/EHU and procedures complied with Spanish and European Union (EU) law.

A total of 4.5 µg of mRNA per day were administered during 3 days. Three instillations of 2.5 µL of the formulations were administered employing a micropipette to one eye in each animal (the other eye was kept as control) at 3 min intervals, twice separated by 12 h.

Forty-eight hours after the last dose, mice were sacrificed and eyeballs were removed, fixed, and sectioned. Immunofluorescence staining was performed to evaluate GFP expression, qualitatively. Tissue sections were examined under a Zeiss LSM800 confocal microscope (ZEISS microscopy, Oberkochen, Germany). Sequential acquisition was used to avoid overlapping of fluorescent emission spectra.

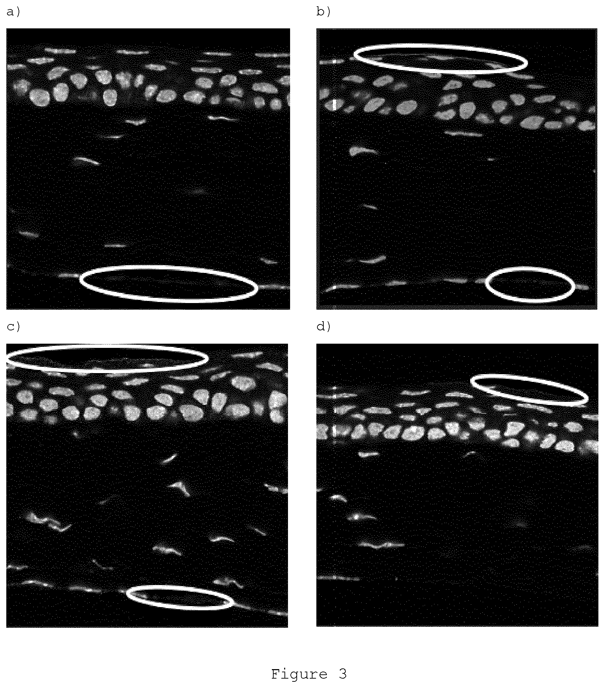

The obtained images show that both SLN-P-HA (Figure 3a) and SLN _Au-P-HA (Figure 3b) were able to transfect corneal tissue; whereas controls confirmed no fluorescence. However, in the cornea of mice treated with the formulation SLN_Au-P-HA, the intensity of fluorescence in the epithelial and endothelial layers, and therefore the amount of protein produced, was higher. A similar pattern of expression was also confirmed for further SLNs according to the invention, and also with DNA as nucleic acid, such as in formulations SLN _Au-P-DX (Figure 3c) or SLN _oro-P-DX (Figure 3d).

## Claims

1. Solid lipid nanoparticle (SLN) comprising:
- a lipid solid at room temperature at the core of the solid lipid nanoparticle;
- a cationic surfactant;
- a non-ionic surfactant;
- gold, wherein the gold is adsorbed onto the surface of the SLN or it is found at a hydrophilic phase of the SLN surrounding the lipid core of the SLN; and
- a nucleic acid.

2. SLN according to claim 1, further comprising a peptide with a net positive charge.

3. SLN according to claim 2, wherein the peptide is protamine.

4. SLN according to any one of claims 1 to 3, further comprising a polysaccharide.

5. SLN according to claim 4, wherein the polysaccharide is dextran or a glycosaminoglycan, preferably the glycosaminoglycan is hyaluronic acid.

6. Suspension comprising a plurality of solid lipid nanoparticles as defined in any one of claims 1 to 5 dispersed in a suspension medium.

7. Process for the preparation of an SLN as defined in any one of claims 1 to 5, comprising:
a) Preparing a suspension of precursor SLN, said precursor SLN comprising:
- a lipid solid at room temperature at the core of the precursor SLN;
- a cationic surfactant; and
- a non-ionic surfactant;
b) Preparing a solution comprising:
- a nucleic acid;
- gold; and
- if required in the SLN to be obtained, a peptide with a net positive charge and/or a polysaccharide;
c) Mixing the suspension of precursor SLN obtained in step (a) with the solution obtained in step (b).

8. Process according to claim 7, wherein step a) comprises:
(i) preparing a solution comprising the lipid solid at room temperature in an organic solvent;
(ii) preparing an aqueous solution comprising the cationic surfactant and the non-ionic surfactant;
(iii) adding the aqueous solution (ii) to the organic solution (i), and subjecting the resulting mixture to stirring until obtaining an emulsion; and
(iv) evaporating the organic solvent.

9. Process according to claim 7, wherein step a) comprises:
(i) melting the lipid solid at room temperature;
(ii) preparing an aqueous solution comprising the cationic surfactant and the non-ionic surfactant;
(iii) adding the aqueous solution (ii) to the melted lipid (i), and subjecting the resulting mixture to stirring until obtaining an emulsion; and
(iv) optionally, subjecting the emulsion (iii) to a homogenization process with a pressure of at least 30 psi.

10. Process for the preparation of an SLN as defined in any one of claims 1 to 5, comprising:
a) Preparing a suspension of precursor gold SLN, said precursor gold SLN comprising:
- a lipid solid at room temperature at the core of the precursor gold SLN;
- a cationic surfactant;
- a non-ionic surfactant; and
- gold;
b) Preparing a solution comprising:
- a nucleic acid; and
- if required in the SLN to be obtained, a peptide with a net positive charge and/or a polysaccharide;
c) Mixing the suspension of precursor gold SLN obtained in step (a) with the solution obtained in step (b).

11. Process according to claim 10, wherein step a) comprises:
(i) preparing a solution comprising the lipid solid at room temperature in an organic solvent;
(ii) preparing an aqueous solution comprising the cationic surfactant, the non-ionic surfactant and the gold;
(iii) adding the aqueous solution (ii) to the organic solution (i), and subjecting the resulting mixture to stirring until obtaining an emulsion; and
(iv) evaporating the organic solvent.

12. Process according to claim 10, wherein step a) comprises:
(i) melting the lipid solid at room temperature;
(ii) preparing an aqueous solution comprising the cationic surfactant, the non-ionic surfactant and the gold;
(iii) adding the aqueous solution (ii) to the melted lipid (i), and subjecting the resulting mixture to stirring until obtaining an emulsion; and
(iv) optionally, subjecting the emulsion (iii) to a homogenization process with a pressure of at least 30 psi.

13. Pharmaceutical composition comprising a plurality of SLN as defined in any one of claims 1 to 5, or a suspension as defined in claim 6, and a pharmaceutically acceptable excipient.

14. SLN as defined in any one of claims 1-5, or suspension as defined in claim 6, or pharmaceutical composition as defined in claim 13, for use in gene therapy.

## Patentansprüche

1. Feste Lipid-Nanopartikel (SLN), umfassend:
- einen Lipidfeststoff bei Raumtemperatur im Kern der festen Lipid-Nanopartikel;
- ein kationisches Tensid;
- ein nichtionisches Tensid;
- Gold, wobei das Gold an der Oberfläche der SLN adsorbiert ist oder sich in einer hydrophilen Phase der SLN befindet, die den Lipidkern der SLN umgibt; und
- eine Nukleinsäure.

2. SLN gemäß Anspruch 1, das ferner ein Peptid mit einer positiven Nettoladung umfasst.

3. SLN gemäß Anspruch 2, wobei das Peptid Protamin ist.

4. SLN gemäß einem der Ansprüche 1 bis 3, das ferner ein Polysaccharid umfasst.

5. SLN gemäß Anspruch 4, wobei das Polysaccharid Dextran oder ein Glykosaminoglykan ist, vorzugsweise ist das Glykosaminoglykan Hyaluronsäure.

6. Suspension, die eine Vielzahl von festen Lipid-Nanopartikeln, wie in einem der Ansprüche 1 bis 5 definiert, umfasst, die in einem Suspensionsmedium dispergiert sind.

7. Verfahren zur Herstellung eines SLN gemäß einem der Ansprüche 1 bis 5, umfassend:
a) Herstellen einer Suspension eines Vorläufer-SLN, wobei das Vorläufer-SLN umfasst:
- einen Lipidfeststoff bei Raumtemperatur im Kern des Vorläufer-SLN;
- ein kationisches Tensid; und
- ein nichtionisches Tensid;
b) Herstellen einer Lösung, umfassend:
- eine Nukleinsäure;
- Gold; und
- falls in dem zu erhaltenen SLN erforderlich, ein Peptid mit einer positiven Nettoladung und/oder ein Polysaccharid;
c) Mischen der in Schritt (a) erhaltenen Suspension von Vorläufer-SLN mit der in Schritt (b) erhaltenen Lösung.

8. Verfahren nach Anspruch 7, wobei Schritt a) umfasst:
(i) Herstellen einer Lösung, die den Lipidfeststoff bei Raumtemperatur in einem organischen Lösungsmittel umfasst;
(ii) Herstellen einer wässrigen Lösung, die das kationische Tensid und das nichtionische Tensid umfasst;
(iii) Zugeben der wässrigen Lösung (ii) zu der organischen Lösung (i) und Rühren der resultierenden Mischung, bis eine Emulsion erhalten wird; und
(iv) Verdampfen des organischen Lösungsmittels.

9. Verfahren nach Anspruch 7, wobei Schritt a) umfasst:
(i) Schmelzen des Lipidfeststoffes bei Raumtemperatur;
(ii) Herstellen einer wässrigen Lösung, die das kationische Tensid und das nichtionische Tensid umfasst;
(iii) Zugeben der wässrigen Lösung (ii) zu dem geschmolzenen Lipid (i) und Rühren der resultierenden Mischung, bis eine Emulsion erhalten wird; und
(iv) optional, Unterziehen der Emulsion (iii) einem Homogenisierungsprozess mit einem Druck von mindestens 30 psi.

10. Verfahren zur Herstellung eines SLN gemäß einem der Ansprüche 1 bis 5, umfassend:
a) Herstellen einer Suspension eines Vorläufer-Gold-SLN, wobei das Vorläufer-Gold-SLN umfasst:
- einen Lipidfeststoff bei Raumtemperatur im Kern des Vorläufer-Gold-SLN;
- ein kationisches Tensid;
- ein nichtionisches Tensid; und
- Gold;
b) Herstellen einer Lösung, die umfasst:
- eine Nukleinsäure; und
- falls in dem zu erhaltenen SLN erforderlich, ein Peptid mit einer positiven Nettoladung und/oder ein Polysaccharid;
c) Mischen der in Schritt (a) erhaltenen Suspension des Vorläufer-Gold-SLN mit der in Schritt (b) erhaltenen Lösung.

11. Verfahren nach Anspruch 10, wobei Schritt a) umfasst:
(i) Herstellen einer Lösung, die den Lipidfeststoff bei Raumtemperatur in einem organischen Lösungsmittel umfasst;
(ii) Herstellen einer wässrigen Lösung, die das kationische Tensid, das nichtionische Tensid und das Gold umfasst;
(iii) Zugeben der wässrigen Lösung (ii) zu der organischen Lösung (i) und Rühren der resultierenden Mischung, bis eine Emulsion erhalten wird; und
(iv) Verdampfen des organischen Lösungsmittels.

12. Verfahren nach Anspruch 10, wobei Schritt a) umfasst:
(i) Schmelzen des Lipidfeststoffes bei Raumtemperatur;
(ii) Herstellen einer wässrigen Lösung, die das kationische Tensid, das nichtionische Tensid und das Gold umfasst;
(iii) Zugeben der wässrigen Lösung (ii) zu dem geschmolzenen Lipid (i) und Rühren der resultierenden Mischung, bis eine Emulsion erhalten wird; und
(iv) optional, Unterziehen der Emulsion (iii) einem Homogenisierungsprozess mit einem Druck von mindestens 30 psi.

13. Pharmazeutische Zusammensetzung, umfassend eine Vielzahl von SLN gemäß einem der Ansprüche 1 bis 5 oder eine Suspension gemäß Anspruch 6 und einen pharmazeutisch akzeptablen Hilfsstoff.

14. SLN gemäß einem der Ansprüche 1 bis 5 oder Suspension gemäß Anspruch 6 oder pharmazeutische Zusammensetzung gemäß Anspruch 13 zur Verwendung in der Gentherapie.

## Revendications

1. Nanoparticule lipidique solide (SLN) comprenant :
- un solide lipidique à température ambiante dans le noyau de la nanoparticule lipidique solide ;
- un tensioactif cationique ;
- un tensioactif non ionique ;
- de l'or, dans laquelle l'or est adsorbé à la surface de la SLN ou se trouve dans une phase hydrophile de la SLN entourant le noyau lipidique du SLN ; et
- un acide nucléique.

2. SLN selon la revendication 1, comprenant en outre un peptide avec une charge nette positive.

3. SLN selon la revendication 2, dans lequel le peptide est la protamine.

4. SLN selon l'une quelconque des revendications 1 à 3, comprenant en outre un polysaccharide.

5. SLN selon la revendication 4, dans lequel le polysaccharide est du dextrane ou un glycosaminoglycane, de préférence le glycosaminoglycane est de l'acide hyaluronique.

6. Suspension comprenant une pluralité de nanoparticules lipidiques solides telles que définies dans l'une quelconque des revendications 1 à 5, dispersées dans un milieu de suspension.

7. Procédé pour la préparation d'une SLN telle que définie dans l'une quelconque des revendications 1 à 5, comprenant :
a) préparer une suspension de précurseur de SLN, ledit précurseur de SLN comprenant :
- un solide lipidique à température ambiante dans le noyau du précurseur de SLN ;
- un tensioactif cationique ; et
- un tensioactif non ionique ;
b) préparer une solution comprenant :
- un acide nucléique ;
- de l'or ; et
- si nécessaire dans la SLN à obtenir, un peptide avec une charge nette positive et/ou un polysaccharide ;
c) mélanger la suspension de précurseur de SLN obtenue à l'étape (a) avec la solution obtenue à l'étape (b).

8. Procédé selon la revendication 7, dans lequel l'étape a) comprend :
(i) préparer une solution comprenant le solide lipidique à température ambiante dans un solvant organique ;
(ii) préparer une solution aqueuse comprenant le tensioactif cationique et le tensioactif non ionique ;
(iii) ajouter la solution aqueuse (ii) à la solution organique (i) et soumettre le mélange obtenu à une agitation jusqu'à obtention d'une émulsion ; et
(iv) évaporer le solvant organique.

9. Procédé selon la revendication 7, dans lequel l'étape a) comprend :
(i) faire fondre le solide lipidique à température ambiante ;
(ii) préparer une solution aqueuse comprenant le tensioactif cationique et le tensioactif non ionique ;
(iii) ajouter la solution aqueuse (ii) au lipide fondu (i) et soumettre le mélange obtenu à une agitation jusqu'à l'obtention d'une émulsion ; et
(iv) éventuellement, soumettre l'émulsion (iii) à un procédé d'homogénéisation à une pression d'au moins 30 psi.

10. Procédé pour la préparation d'une SLN telle que définie dans l'une quelconque des revendications 1 à 5, comprenant :
a) préparer une suspension de précurseur de SLN à base d'or, ledit précurseur de SLN à base d'or comprenant :
- un solide lipidique à température ambiante dans le noyau du précurseur de SLN à base d'or;
- un tensioactif cationique ;
- un tensioactif non ionique ; et
- de l'or ;
b) préparer une solution comprenant :
- un acide nucléique ; et
- si nécessaire dans la SLN à obtenir, un peptide avec une charge nette positive et/ou un polysaccharide ;
c) mélanger la suspension de précurseur de SLN à base d'or obtenue à l'étape (a) avec la solution obtenue à l'étape (b).

11. Procédé selon la revendication 10, dans lequel l'étape a) comprend :
(i) préparer une solution comprenant le solide lipidique à température ambiante dans un solvant organique ;
(ii) préparer une solution aqueuse comprenant le tensioactif cationique, le tensioactif non ionique et l'or ;
(iii) ajouter la solution aqueuse (ii) à la solution organique (i), et soumettre le mélange résultant à une agitation jusqu'à l'obtention d'une émulsion ; et
(iv) évaporer le solvant organique.

12. Procédé selon la revendication 10, dans lequel l'étape a) comprend :
(i) faire fondre le solide lipidique à température ambiante ;
(ii) préparer une solution aqueuse comprenant le tensioactif cationique, le tensioactif non ionique et l'or ;
(iii) ajouter la solution aqueuse (ii) au lipide fondu (i) et soumettre le mélange résultant à une agitation jusqu'à l'obtention d'une émulsion ; et
(iv) éventuellement, soumettre l'émulsion (iii) à un procédé d'homogénéisation à une pression d'au moins 30 psi.

13. Composition pharmaceutique comprenant une pluralité de SLN telle que définie dans l'une quelconque des revendications 1 à 5, ou une suspension telle que définie dans la revendication 6, et un excipient pharmaceutiquement acceptable.

14. SLN telle que définie dans l'une quelconque des revendications 1 à 5, ou suspension telle que définie dans la revendication 6, ou composition pharmaceutique telle que définie dans la revendication 13, pour usage en thérapie génique.
